# EUROPEAN PATENT APPLICATION

(11) **EP 1 186 661 A1**
(43) Date of publication of application: **13.03.2002**
(21) Application number: 00402436.0
(22) Date of filing: 05.09.2000
(51) Int. Cl.: C12N 15/12, C07K 14/705, C12Q 1/68, G01N 33/48, G01N 33/53

(54) **Identification of the major capacitative calcium channel in antigen presenting cells and uses thereof**

(71) Applicant: WARNER-LAMBERT COMPANY, Morris Plains New Jersey 07950 (US)
(72) Inventor: Allen, Janet, 92190 Meudon (FR); Grentzmann, Guido, 91370 Verrières Le Buisson (FR); Fink, Michael, 94260 Fresnes (FR); Schindler, Véronique, 75005 Paris (FR); Bloes, Carole, 91400 Orsay (FR)
(74) Representative: Becker, Philippe

(57) **Abstract**

The present invention relates to the identification of the major capacitative calcium channel of cells of the immune system, and its use to modulate the activity of immune cells *in vitro, ex vivo* or *in vivo.* This invention more specifically discloses that TrpC8, a Mlsnl gene product, represents the major capacitative calcium channel of macrophage cells. This invention more particularly resides in the use of a Mlsnl gene, gene product or transcription promoter for the screening of a compound that modulates the activity of an antigen presenting cell, as well as in the use of a Mlsnl gene, gene product or transcription promoter to identify new (known or unknown) targets for immune response modulation.

## Description

### FIELD OF INVENTION

The present invention relates to the identification of the major capacitative calcium channel of cells of the immune system, and its use to modulate the activity of immune cells *in vitro, ex vivo* or *in vivo*. This invention more specifically discloses that TrpC8, an Mlsnl gene product, represents the major capacitative calcium channel of immune cells such as macrophages, monocytes, T-cells, B-cells and mast-cells. This invention is the first proven identification of a gene expressing a capacitative calcium channel in immune cells, and can be used in various compositions and methods for modulating an immune response in a subject. The present invention can further be used to develop specific immune system activity altering drugs as well as to identify new, known or unknown immune system related targets and gene pathways.

### BACKGROUND OF THE INVENTION

Regulated entry of calcium across the plasma membrane is an essential signaling mechanism, implicated in phenomena like exocytosis, contraction, gene expression, and cell differentiation (1). Store operated channels (SOCs), defined as channels that open in response to depletion of intra-cellular calcium stores, represent one of the most ubiquitous mechanisms for triggering calcium influx in non-excitable cells.

A large number of cell surface receptors are coupled through G-proteins or tyrosine kinases to the activation of phospholipase C (PLC) and consequently the generation of inositol 1,4,5-triphosphate (IP3) (2). Increased IP3, acting on its receptor (3) results in an increase of intra-cellular calcium ([Ca²⁺]i) through depletion of intra-cellular calcium stores. How depletion of intra-cellular calcium stores triggers Ca²⁺entry through the cell membrane is still controversial. The simplest mechanism would involve a direct action of IP3, or of the IP3 receptor, on Ca²⁺ channels in the plasma membrane. On the other hand depletion of stores might send an activating signal of some kind to channels in the plasma membrane (4). Plasma membrane Ca²⁺ channel activation results in a small inward current in parallel with a sustained raise in [Ca²⁺]i. Such a current has been shown to be induced by intra-cellular dialysis with IP3 in rat mastcells (5) or by Thapsigargin-activation of Jurkat T-cells (6). Thapsigargin (TG), a plant derived sesquiterpene lactone, depletes IP3-sensitive stores through its ability to inhibit Ca²⁺-ATPases (7). Several groups have shown that TG depletion of intracellullar calcium stores in T-cells activates a Ca²⁺ influx that was similar to and not additive with that evoked by T-Cell Receptor (TCR) stimulation (8-10). This Ca²⁺current, later identified as an Icrac current (Ca²⁺ release activated Ca²⁺ current), lacks voltage dependent gating and is characterized by an inward rectifying current-voltage relation, blockage by Ni²⁺ and Cd²⁺, and feedback inhibition by Ca²⁺.

SOCs have a high diversity. The essential defining feature of these channels is that they are activated by a variety of stimuli that deplete intra-cellular stores such as agonists to phosphoinositide-linked receptors, intra-cellular IP3, Ca²⁺ ionophores, inhibitors of SERCA-type Ca²⁺ATPases, and intra-cellular dialysis with buffered solutions containing low concentrations of Ca²⁺ ([Ca²⁺]<50 nM) (1). IP3 activation of SOCs implies that the channel is not directly coupled to the receptor and artificial store depletion activation indicates that SOC activation responds to store Ca²⁺ depletion after IP3-receptor binding than to IP3 itself. SOCs are generally described as lacking voltage dependent gating.

Icrac can be distinguished from other SOCs by its extremely high selectivity for Ca²⁺ over monovalent cations and its small unitary conductance (11). The unitary cord conductance of the channel has been estimated between 10 and 24 fS in Jurkat cells (12). This is more than 100-fold lower than other known Ca²⁺ channels.

Calcium entry through Icrac has been shown to accelerate the rate of exocytosis in mast cells. It inhibits adenylyl cyclase type VI, but activates adenylyl cyclase type I. Icrac activity might be regulated either directly or indirectly through the concentration of intra-cellular Ca²⁺ (13). An increase in the intra-cellular calcium ion concentration controls a diverse range of cell functions, including adhesion, mobility, gene expression and proliferation and can occur as single transients, repetitive oscillations or a sustained plateau (14). Two recent reports have suggested that the amplitude, duration and frequency of a Ca²⁺ signal in cells of the immune system control differential activation of proinflammatory transcriptional regulators NF-κB, JNK and NF-AT (15; 16). Thus Icrac could play a crucial role in the activation of cells of the immune system. An impressive indication to this is the observation that Icrac current was completely absent from T-cells of a patient suffering from a primary immunodeficiency (17). The identification of the Icrac channel would therefore represent an important step forward in determining the pathology of Icrac in disease states. This target could be used as a basis of screens to identify new therapeutic compounds such as immune system suppressors, including anti-allergic or anti-inflammatory compounds, or immune system activators for the treatment of immuno degenerative disorders.

### SUMMARY

The present invention discloses the identification of the major Ca²⁺ channel present in cells of the immune system, more specifically in macrophages.

More particularly, the inventors have identified the presence of a novel calcium pore sequence within the product of the Mlsnl gene, and this calcium pore is part of the major capacitative calcium channel in immune system cells, more specifically in macrophages.

The present invention shows that a product of the Mlsnl gene is expressed in the U937 cells (monocyte like) specifically after 48h differentiation to a more macrophage like cell type and that Mlsnl regulates capacitative calcium entry in these cells. PCR subtraction experiments show that Mlsnl gene expression is several times upregulated, which is in concordance with the previously established differential increases of the Icrac phenotype during this differentiation step (18).

This invention also shows that particular Mlsnl-specific anti-sense nucleic acids down regulate calcium entry in U937 cells differentiated to a macrophage phenotype. This is of particular importance since it is the first time that a sequence specific tool has succeeded to inhibit the Icrac phenotype, thereby associating a given gene to the Ca²⁺ pore gene expression.

This invention represents the first discovery and identification of an Icrac channel in immuno-modulatory cells, more specifically in macrophages. This invention provides a means to identify new targets for the screening of immuno-modulators.

The described invention further shows, the nucleic acid sequence of Icrac-Mlsnl transcriptional promoter elements 5'to the putative Mlsnl start codon.

An object of the present invention resides in the use of a Icrac-Mlsnl gene, gene product(s) and transcription promoters for the screening of compounds that modulate the activity of an Icrac expressing immune cell, more specifically of an antigen presenting cell, most specifically of a compound that modulates the activity of macrophages.

Further objects of the present invention include Icrac-Mlsnl peptides, nucleic acids, vectors, and recombinant cells, which can be used for therapeutic or diagnostic approaches, or as targets for development of drug compounds or candidates in various screening assays methods of identification of additional (known or unknown) targets or gene pathways involved in immune system activity.

### BRIEF DESCRIPTION OF THE FIGURES

Figure 1: Amino acid sequence of Icrac-Mlsnl with predicted secondary structure.
Where;
<<N-TERMINUS>> represents the N-terminal domain
_TMD1 to 6 represent the transmembrane domains
--EXL1 to 3-- represents the extra-cellular loop
--INL1and 2-- represent the intra-cellular loops
<<C-TERMINUS>> represents the C-terminal domain

Figure 2: Represents the analysis on agarose of PCR products obtained with Mlsnl-specific primer pairs from differentiated or undifferentiated U937 macrophage cells.

Figure 3: Illustrates the Mlsnl(TrpC8) expression analysis by cDNA subtraction.
1 : dbcAMP differentiated U937 subtracted by undifferentiated U937.
2 : unsubtracted dbcAMP differentiated U937 control.
3 : undifferentiated U937 subtracted by dbcAMP differentiated U937.
4 : unsubtracted undifferentiated U937 control.
5 : lkb ladder
   PCR have been carried out on subtraction DNA fragments using "Not 1a" and "Not 2b" Mlsnl specific primers.

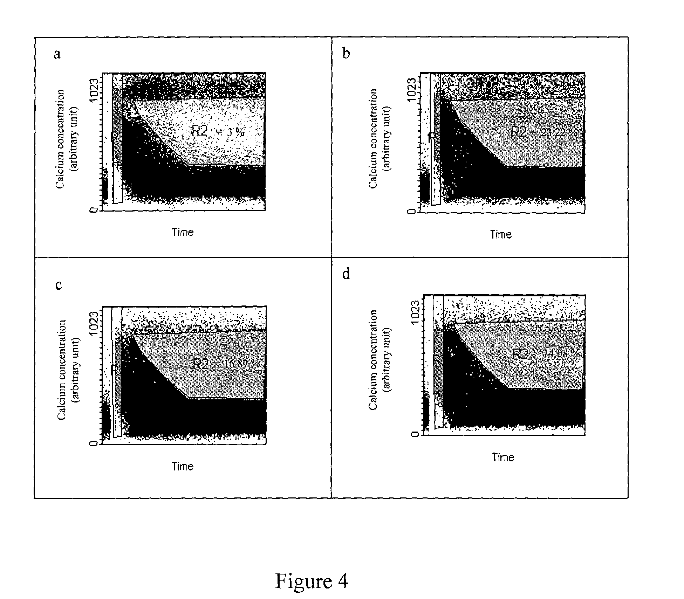

Figure 4: Down regulation of calcium entry (Icrac phenotype) during dbcAMP differentiation of U937 cells using Mlsnl anti-sense - Calcium flux analysis using Indo-l and flow cytometry
a : Undifferentiated U937
b : dbcAMP differentiated U937 treated with 10 µM scrambled oligonucleotide
c : dbcAMP differentiated U937 treated with 1 µM Mlsnl anti-sense
d : dbcAMP differentiated U937 treated with 10 µM Mlsnl anti-sense

### DETAILED DESCRIPTION OF THE INVENTION

An object of the present invention resides in the identification of functional components of Icrac-Mlsnl. The polypeptide structure of Icrac-Mlsnl has been determined. Nucleic acid sequences and peptides which encode the N- and C-terminal regions have been identified. Also, nucleic acid sequences and peptides which encode transmembrane domains and intra- and extra-cellular loop regions have been identified. Crucially, nucleic acid and peptide sequences which encode the Icrac-Mlsnl calcium pore region have been identified. The above nucleic acid and peptide sequences offer vital target molecules for the development of therapeutic molecules to combat disorders associated with aberrant Icrac function.

Furthermore, the inventors have identified putative transcription promoter elements 5' to the nucleic acid encoding Methionine-1 start codon of Icrac-Mlsnl.

Another object of this invention resides, generally, in the use of a Icrac-Mlsnl gene, gene product, fragments thereof, or transcriptional promoter elements for the screening of compound(s) that modulate the activity of an immune cell, more specifically for the screening of a compound that modulates the activity of monocytes, macrophages, T-cells, B-cells and mast-cells *in vitro, ex vivo* or *in vivo,* most specifically for the screening of compounds that modulate the activity of macrophages *in vitro, ex vivo* or *in vivo.* This invention also relates to particular Icrac-Mlsnl polypeptides and nucleic acids, methods of screening of immuno-modulators.

### Other aspects of the present invention relate to:

Methods of screening for immuno-modulatory compounds, comprising contacting a test compound with a Icrac-Mlsnl gene or a Icrac-Mlsnl gene product or Icrac-Mlsnl transcription promoter element(s) and determining the ability of said test compound to interact with the Icrac-Mlsnl gene or a Icrac-Mlsnl gene product or Icrac-Mlsnl transcriptional promoter element(s).

Methods of screening for identification of new (known or unknown) immuno-modulatory targets after modulation of the Icrac-Mlsnl gene, Icrac-Mlsnl gene product activity or transcription promoter activity.

### Further aspects of the present invention relate to:

The use of Icrac-Mlsnl gene, gene products or nucleic acids to modulate the activity of Icrac-Mlsnl in immune cells *in vitro, ex vivo or in vivo.*

Within the context of the present invention, the term Mlsnl gene designates a nucleotide sequence encoding the Mlsnl protein, any nucleotide sequence encoding a fragment of the Mlsnl protein, or any variants or homologs thereof. Variants or homologs designate more particularly any naturally-existing human genes that would exhibit sequence variation(s) resulting from polymorphism(s), mutation(s), or other alteration(s) as compared to the above sequence, without depriving the property of the encoded protein to function as an Icrac channel. Homologs or variants also include any recombinant DNA molecule, such as a cDNA molecule, encoding a Mlsnl protein. Further variants also include any sequence that would hybridize with the above nucleic acid sequences under stringent conditions and encode a functionally active Icrac channel. Nucleotide sequences encoding fragments of the Mlsnl protein which represent a biological relevant region of this protein such as the N- and C- terminal domains, the transmembrane domains, the intra and extra-cellular loops and the predicted calcium pore as well as the polypeptides representing these biologically relevant regions are also included.

### As an illustrative embodiment, stringent hybridization conditions can be defined as follows:

The hybridization step is conducted at 65°C in the presence of 6 x SSC buffer, 5 x Denhardt's solution, 0.5 % SDS and 100µg/ml of salmon sperm DNA.

### The hybridization step is followed by four washing steps:

- two washings during 5 minutes, preferably at 65°C in a 2 x SSC and 0.1% SDS buffer;
- one washing during 30 minutes, preferably at 65°C in a 2 x SSC and 0.1% SDS buffer;
- one washing during 10 minutes, preferably at 35°C in a 0.1 x SSC and 0.1% SDS buffer,
   it being understood that the hybridization conditions defined above are suitable for nucleic acids of approximately twenty nucleotides in length and that these conditions may be also adapted for shorter or longer nucleic acids, according to techniques well known in the art, for example those described by Sambrook et al. (1989).

A Mlsnl gene product designates, in accordance with the present invention, polypeptides (or proteins) encoded by a Mlsnl gene as described above. The Mlsnl gene product may comprise several polypeptides, resulting from alternative splicings of a Mlsnl gene during transcription. More specific Mlsnl gene products of this invention are encoded by a Mlsnl gene as described above and/or comprise the amino acid sequence of Mlsnl, such as that of SEQ ID N°1.

Also within the context of the present invention, the term Icrac-Mlsnl transcription promoter(s) designates nucleotide sequence(s) encoding Icrac-Mlsnl transcription promoter element(s), nucleotide sequence encoding a fragment thereof, or any variants or homologs thereof. Variants or homologs designate more particularly any human transcription promoter elements that would exhibit sequence variation(s) resulting from polymorphism(s), mutation(s), or other alteration(s) as compared to the above sequence, without depriving the property of the nucleic acid sequences to function as an Icrac transcription promoter. Further variants also include any sequence that would hybridize with the above nucleic acid sequences under stringent conditions and encode a functionally active transcription promoter.

A Icrac-Mlsnl transcription promoter element designates, in accordance with the present invention, encoded by a Icrac-Mlsnl nucleic acid sequence as described above. The transcription promoter elements is further characterized by being located 5' the Icrac-Mlsnl start codon. More specific Icrac-Mlsnl transcription promoter elements of this invention are encoded by Icrac-Mlsnl nucleic acid sequences of SEQ ID N°41, 42 or 43.

### NUCLEOTIDE AND AMINO ACID SEQUENCE ENCODING Icrac-Mlsnl

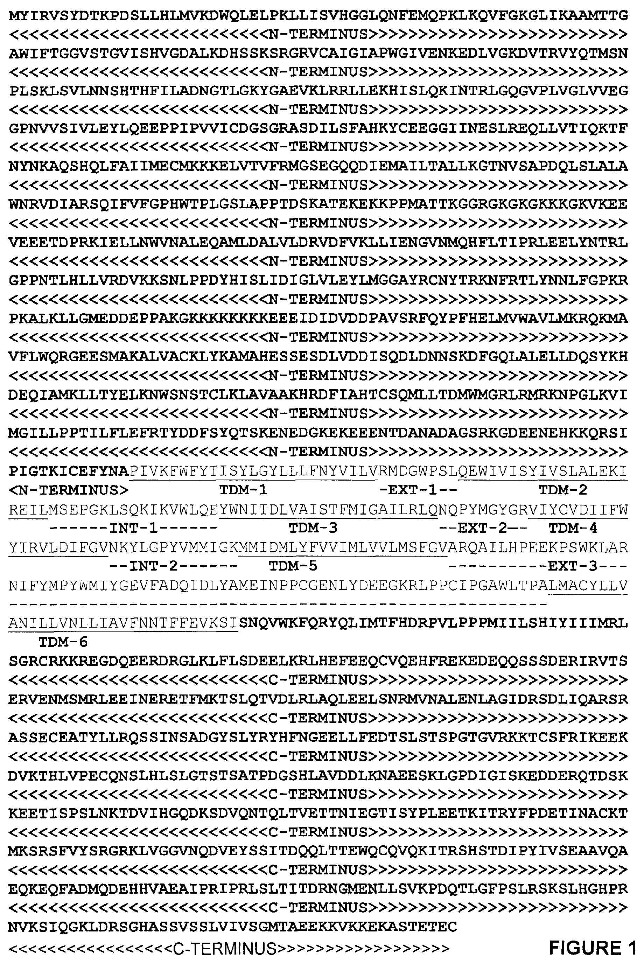

The human Mlsnl cDNA has been described in US Patent 5,674,739 as FOHY030. This gene is described as being expressed specifically in certain tumor cells and not in healthy tissues and was thus characterized as involved in tumor progression. The sequence of human Mlsnl cDNA is also available from Genbank (accession number AF071787). The published nucleic acid sequence encoding the open reading frame of human Mlsnl is predicted to be 4602 nucleic acids in length (including a stop codon). This nucleotide sequence is shown in SEQ ID N°23. This published Mlsnl gene encodes a linear polypeptide of 1533 amino acids. The predicted amino acid sequence of this Mlsnl is shown in SEQ ID N°1 and figure 1. The present invention stems from the discovery that expression of an Icrac-Mlsnl gene product is essential for a functional capacitative calcium channel in immune- cells such as monocytes, macrophages, T-cells, B-cells and mast-cells, specifically in antigen presenting cells, more specifically in macrophages. Considering the implication of calcium influx in regulating various cell activities, this invention provides additional means in designing immuno-modulatory compounds. More particularly, this invention now provides a novel target for screening immuno-modulatory compounds and deciphering immune activation gene pathways.

Therefore, a first object of the invention is the use of any purified or isolated nucleic acid encoding a human Icrac-Mlsnl or a sequence complementary thereto, wherein, the encoding nucleic acid encodes a polypeptide having the following characteristics;
(1) the N-terminal region is encoded by a consecutive sequence of at least 75 nucleotides, preferably at least 300 nucleotides, more preferably at least 750 nucleotides , most preferably 1500 nucleotides, located 5' with respect to nucleotidesencoding the transmembrane and C- terminal regions of the Icrac-Mlsnl protein. More particularly the sequence is selected within nucleotides1 to 2268 of SEQ ID N°23,
(2) a transmembrane region encoding six membrane spanning domains, encoded by a consecutive sequence of at least 150 nucleotides, preferably at least 300 nucleotides, more preferably at least 750 nucleotides, located 3' with respect to the nucleotidesencoding the N-terminal region and 5' with respect to nucleotidesencoding the C-terminal region of Icrac-Mlsnl. More particularly the sequence is selected within the nucleotides2271 to 3045 of SEQ ID N°23, and
(3) a C-terminal region encoded by a consecutive sequence of at least 75 nucleotides, preferably at least 150 nucleotides, more preferably at least 600 nucleotides located 3' with respect to nucleotidesencoding the N-terminal region and transmembrane region of Icrac-Mlsnl. More particularly selected within nucleotides 3048 to 4599 of SEQ ID N°23, for the screening of Icrac-Mlsnl modulators, more specifically Icrac modulators. A preferred nucleic acids encoding Icrac-Mlsnl is that of SEQ ID N°23.

A further object of the invention consists of the use of any purified or isolated nucleic acid having at least 80%, preferably 90%, more preferably 95% and most preferably 98% nucleotide identity with the nucleotide sequence of SEQ ID N°23 or a sequence complementary thereto, for the screening of Icrac-Mlsnl modulators, more specifically Icrac modulators. Also encompassed by the present invention is the use of any nucleic acid that hybridizes, under stringent condition, with SEQ ID N°23, its complementary strand, or a portion thereof, and encodes a polypeptide with calcium channel activity.

A second object of the invention is the use of any purified or isolated peptide encoding a human Icrac-Mlsnl or fragment thereof, wherein, the peptide has the following characteristics;
(1) the N-terminal region comprises consecutive sequence of at least 25 amino acids, preferably at least 100 amino acids, more preferably at least 250 amino acids, most preferably 500 amino acids, located N-terminal with respect to the transmembrane and C-terminal regions of Icrac-Mlsnl protein. More particularly the sequence is selected within the amino acids 1 to 756 of SEQ ID N°1,
(2) a transmembrane region forming six membrane spanning domains, comprising a consecutive sequence of at least 50 amino acids, preferably at least 100 amino acids, more preferably at least 250 amino acids, C-terminal with respect to the N-terminal region, and N-terminal with respect to the C-terminal region of Icrac-Mlsnl. More particularly the sequence is selected within amino acids 757 to 1015 of SEQ ID N°1, and
(3) a C-terminal region comprising a consecutive sequence of at least 25 amino acids, preferably at least 50 amino acids, more preferably at least 200 amino acids, located C-terminal with respect to the N-terminal and transmembrane regions of Icrac-Mlsnl. More particularly the sequence is selected within amino acids 1016 to 1533 of SEQ ID N°1, for the screening of Icrac modulators, more specifically Icrac-Mlsnl modulators. Preferred amino acids encoding Icrac-Mlsnl is that of SEQ ID N°1.

A further object of the invention consists of the use of any purified or isolated peptide having at least 90%, preferably 95%, more preferably 98% and most preferably 99% amino acid identity with the sequence of SEQ ID N°1 or fragment thereof, for the screening of Icrac-Mlsnl modulators, more specifically Icrac modulators.

Another object of the invention is a purified or isolated nucleic acid sequence encoding a polypeptide, wherein said nucleic acid encodes a polypeptide having at least 90%, preferably 95%, more preferably 98% and most preferably 99% sequence identity with the polypeptide of SEQ ID N°1.

Furthermore, the discovery that Icrac function is modulated by Icrac-Mlsnl provides a means to correlate the severity of an Icrac associated disorder with the levels of expression in immune cells.

A third object of the invention is the use of any purified or isolated nucleic acid encoding a human Icrac-Mlsnl or a sequence complementary thereto, wherein, the encoding nucleic acid encodes a polypeptide having the following characteristics;
(1) the N-terminal region is encoded by a consecutive sequence of at least 75 nucleotides, preferably at least 300 nucleotides, more preferably at least 750 nucleotides, most preferably 1500 nucleotides, located 5' with respect to nucleotides encoding the transmembrane and C- terminal regions of Icrac-Mlsnl protein. More particularly the sequence is selected within nucleotides 1 to 2268 of SEQ ID N°23,
(2) a transmembrane region encoding six membrane spanning domains, encoded by a consecutive sequence of at least 150 nucleotides, preferably at least 300 nucleotides, more preferably at least 750 nucleotides, located 3' with respect to the nucleotides encoding the N-terminal region and 5' with respect to nucleotides encoding the C-terminal region of Icrac-Mlsnl. More particularly the sequence is selected within nucleotides 2271 to 3045 of SEQ ID N°23, and
(3) a C-terminal region encoded by a consecutive sequence of at least 75 nucleotides, preferably at least 150 nucleotides, more preferably at least 600 nucleotides, located 3' with respect to nucleotides encoding the N-terminal region and transmembrane region of Icrac-Mlsnl. More particularly the sequence is selected within nucleotides 3048 to 4599 of SEQ ID N°23, for the diagnosis of disorders associated with aberrant Icrac function in immune cells. A preferred nucleic acid encoding Icrac-Mlsnl is that of SEQ ID N°23.

A further object of the invention consists of the use of any purified or isolated nucleic acid having at least 80%, preferably 90%, more preferably 95% and most preferably 98% nucleotide identity with the nucleotide sequence of SEQ ID N°23 or a sequence complementary thereto, for the diagnosis of disorders associated with aberrant Icrac function in immune cells.

A fourth object of the invention is the use of any purified or isolated peptide encoding a human Icrac-Mlsnl or fragment thereof, wherein, the peptide has the following characteristics;
(1) the N-terminal region comprises a consecutive sequence of at least 25 amino acids, preferably at least 100 amino acids, more preferably at least 250 amino acids, most preferably 500 amino acids, located N-terminal with respect to the transmembrane and C- terminal regions of Icrac-Mlsnl protein. More particularly the sequence is selected within amino acids 1 756 of SEQ ID N°1,
(2) a transmembrane region forming six membrane spanning domains, comprising a consecutive sequence of at least 50 amino acids, preferably at least 100 amino acids, more preferably at least 250 amino acids, located C-terminal with respect to the N-terminal region and N-terminal with respect to the C-terminal region of Icrac-Mlsnl. More particularly the sequence is selected within nucleic acids 757 to 1015 of SEQ ID N°1, and
(3) a C-terminal region comprising a consecutive sequence of at least 25 amino acids, preferably at least 50 amino acids, more preferably at least 200 amino acids, located C-terminal with respect to the N-terminal and transmembrane regions of Icrac-Mlsnl. More particularly the sequence is selected from amino acids 1016 to 1533 of SEQ ID N°1, for the diagnosis of disorders associated with aberrant Icrac function in immune cells. A preferred nucleic acids encoding Icrac-Mlsnl is that of SEQ ID N°1.

A further object of the invention consists of the use of any purified or isolated peptide having at least 90%, preferably 95%, more preferably 98% and most preferably 99% amino identity with the sequence of SEQ ID N°1 or fragment thereof, for the screening of Icrac modulators and the diagnosis of disorders associated with aberrant Icrac function in immune cells.

### STRUCTURE OF Icrac-Mlsnl

The inventors have identified biological relevant components of the Icrac-Mlsnl gene and expression products. The inventors performed gene alignment experiments with various subtypes of voltage-gated Ca²⁺ channels, using the Clustal method (MegAlign-DNAstar/Lasergene software package). In particular, the amino acid sequence of the following Trp proteins were compared, in a window of 41 residues between the predicted transmembrane segments 5 and 6 (the corresponding Genbank accession numbers are indicated): Trp, J04844; TRPL calmodulin-binding protein, M88185; TRPC7 transient receptor potential channel 7, NM 003307; Melastatin 1, AF071787 and the genome sequence of the nematode Caenorhabditis elegans, 277132. These experiments allowed the identification of a novel calcium pore sequence in the Icrac-Mlsnl gene. The inventors believe that the active form of Icrac-Mlsnl is a membrane spanning polypeptide. This polypeptide is predicted to possess 6 transmembrane domains (TRD 1-6), and cytoplasmic N- and C-terminal domains. The N-domain, C- domain and TMDs are all connected by 5 peptide loops, two intra-cellular loops and three extra-cellular loops. Extra-cellular loop 3 forming part of the Icrac calcium pore. The TMDs incorporate the following peptides: TRD1, amino acids 757-781 (as shown in figure 1 of the polypeptide sequence with anotations); TMD2, amino acids 790-810 (as shown in figure 1); TMD3, amino acids 828-849 (as shown in figure 1); TMD4, amino acids 859-878 (as shown in figure 1); TMD5 amino acids 892-912 (as shown in figure 1) and TMD6, amino acids 985-1015 (as shown in figure 1). The N-terminal region includes amino acids 1 to 756 as shown in SEQ ID N°2 and 3, and in figure 1. The C-terminal region includes amino acids 1016-1533 as shown in SEQ ID N°4 and 5, and in figure 1. Intra-cellular loop 1 is encoded by amino acids 811-827 as shown in SEQ ID N°6 and 7, and in figure 1 and intra-cellular loop 2 is encoded by amino acids 879-891 as shown in SEQ ID N°8 and 9 and figure 1. Extra-cellular loop 1 is encoded by amino acids 782-789 as shown in SEQ ID N°10 and 11, and in figure 1. Extra-cellular loop 2 is encoded by amino acids 850-858 as shown in SEQ ID N°12 and 13 and in figure 1. Extra-cellular loop 3 is encoded by amino acids 913-984 as shown in SEQ ID N°14 to 17 and figure 1. The inventors believe that extra-cellular loop 3 plays a role in forming the calcium channel pore. Furthermore, the inventors believe that the 23 amino acid (amino acids 922-944) peptide sequence of SEQ ID N°16 which is predicted to be part of the TrpC8 calcium pore is essential for the function of Icrac.

The inventors believe that intra-cellular and extra-cellular exposed surfaces of Icrac-Mlsnl as well as the calcium pore provide vital target molecules for the development of therapeutic compounds to modulate Icrac. These surfaces include amino acids encoding the N- and C-terminal regions, as well as amino acids which encode loops connecting the transmembrane domains. Crucially, the ascribing of Icrac function to Icrac-Mlsnl has enabled the inventors to identify a calcium pore within the transmembrane region, and an external peptide loop. This loop is thought to directly modulate calcium flux in Icrac. Both the calcium pore and associated loop hold special significance in the development of therapeutics for the treatment of aberrant Icrac function. Furthermore, peptides of Icrac-Mlsnl allow the development of diagnostic tools to assay for Icrac function and levels in immune cells.

Likewise the nucleic acids which encode the said peptides offer opportunities to identify modulators of Icrac, and the development of diagnostic tools.

### Peptides of Icrac-Mlsnl

The inventors believe that the N-terminal cytoplasmic region of Icrac-Mlsnl could play a role in the translocation of the Icrac-Mlsnl polypeptide from the cytoplasm to the cell membrane.

Therefore, an object of the invention is a purified or isolated human Icrac-Mlsnl N-terminal region, wherein the N-terminal region comprises a consecutive sequence of at least 25 amino acids, preferably at least 100 amino acids, more preferably at least 250 amino acids and most preferably at least 500 amino acids, located N-terminal of the first transmembrane region of the Icrac-Mlsnl protein. Preferred is a consecutive sequence taken from the amino acid sequences of SEQ ID N°2 or 3. More preferred are the sequences of SEQ ID N°2 or 3.

A further object of the invention resides in a purified or isolated human Icrac-Mlsnl N-terminal region, wherein the N-terminal region has at least 90%, preferably 95%, more preferably 98% and most preferably 99% amino acid identity with any of the peptides of amino acid sequences SEQ ID N°2 or 3, or fragments thereof.

The inventors also believe that the C-terminal region of Icrac-Mlsnl plays a role in the modulation of activity of Icrac-Mlsnl. Therefore, a second object of the invention consists of a purified or isolated human Icrac-Mlsnl C-terminal region, wherein the C-terminal region comprises a consecutive sequence of at least 25 amino acids, preferably at least 50 amino acids, more preferably at least 100 amino acids and most preferably at least 200 amino acids, located C-terminal of the sixth transmembrane region of the Icrac-Mlsnl protein. Preferred is a consecutive sequence taken from the amino acid sequence of SEQ ID N°4 or 5. More preferred is the sequences of SEQ ID N°4 or 5.
A further object of the invention resides in a purified or isolated human Icrac-Mlsnl C-terminal region, wherein the C-terminal region has at least 90%, preferably 95%, more preferably 98% and most preferably 99% amino acid identity with any of the peptides of amino acid sequences SEQ ID N°4 or 5, or fragments thereof.

The inventors believe that peptide encoding intra-cellular loop-1 of Icrac-Mlsnl play an important role in the regulation of Icrac-Mlsnl and its capacity to transduce intra-cellular signals. Therefore, a third object of the invention is a purified or isolated human Icrac-Mlsnl intra-cellular loop-1 region, wherein the intra-cellular loop-1 region comprises a consecutive sequence of at least 7 amino acids, preferably at least 10 amino acids, more preferably at least 15 amino acids, located C-terminal of the second transmembrane region and N-terminal to the third transmembrane region of the Icrac-Mlsnl protein. Preferred is a consecutive sequence taken from the amino acid sequence of SEQ ID N°6 or 7. More preferred are the sequences of SEQ ID N°6 or 7.
A further object of the invention resides in a purified or isolated human Icrac-Mlsnl intra-cellular loop-1 region, wherein the intra-cellular loop-1 region has at least 90%, preferably 95%, more preferably 98% and most preferably 99% amino acid identity with any of the peptides of amino acid sequences SEQ ID N°6 or 7, or fragments thereof.

The inventors believe that peptide encoding intra-cellular loop-2 of Icrac-Mlsnl also play an important role in the regulation of Icrac-Mlsnl and its capacity to transduce intra-cellular signals. Therefore, a fourth object of the invention is a purified or isolated human Icrac-Mlsnl intra-cellular loop-2 region, wherein the intra-cellular loop-2 region comprises a consecutive sequence of at least 7 amino acids, preferably at least 10 amino acids, located C-terminal of the fourth transmembrane region and N-terminal of the fifth transmembrane region of the Icrac-Mlsnl protein. Preferred is a consecutive sequence taken from the amino acid sequences of SEQ ID N°8 or 9. More preferred is the sequences of SEQ ID N°8 or 9.
A further object of the invention is a purified or isolated human Icrac-Mlsnl intra-cellular loop-2 region, wherein the intra-cellular loop-2 region has at least 90%, preferably 95%, more preferably 98% and most preferably 99% amino acid identity with any of the peptides of amino acid sequences SEQ ID N°8 or 9, or fragments thereof.

The inventors believe that the peptides encoding extra-cellular loop 1 of Icrac-Mlsnl may play a crucial role in the modulation of calcium through the Icrac channel. Therefore a fifth object of the invention is purified or isolated human Icrac-Mlsnl extra-cellular loop-1 region, wherein the extra-cellular loop-1 region comprises a consecutive sequence of at least 5 amino acid located C-terminal of the first transmembrane region and N-terminal of the second transmembrane region of the Icrac-Mlsnl protein. Preferred is a consecutive sequence taken from the amino acid sequences of SEQ ID N°10 or 11. More preferred is the sequence of SEQ ID N°10 or 11.
A further object of the invention is purified or isolated human Icrac-Mlsnl extra-cellular loop-1 region, wherein the extra-cellular loop-1 region has at least 90%, preferably 95%, more preferably 98% and most preferably 99% amino acid identity with any of the peptides of amino acid sequences SEQ ID N°10 or 11, or fragments thereof.

The inventors believe that the peptides encoding extra-cellular loop 2 region of Icrac-Mlsnl may also play a crucial role in the modulation of calcium through the Icrac channel. Therefore, a sixth object of the invention is purified or isolated human Icrac-Mlsnl extra-cellular loop-2 region, wherein the extra-cellular loop-2 region comprises a consecutive sequence of at least 5 amino acids, located C-terminal of the third transmembrane region and N-terminal of the fourth transmembrane region of the Icrac-Mlsnl protein. Preferred is a consecutive sequence taken from the amino acid sequence of SEQ ID N°12 or 13. More preferred is the sequence of SEQ ID N°12 or 13
A further object is purified or isolated human Icrac-Mlsnl extra-cellular loop-2 region, wherein the extra-cellular loop-2 region has at least 90%, preferably 95%, more preferably 98% and most preferably 99% amino acid identity with any of the peptides of amino acid sequences SEQ ID N°12 or 13, or fragments thereof.

The inventors believe that extra-extra-cellular loop-3 region, has a function in the modulation of calcium flux in Icrac. Therefore, a seventh object of the invention is a purified or isolated human Icrac-Mlsnl extra-cellular loop-3 region, wherein the extra-cellular loop-3 region comprises a consecutive sequence of at least 7 amino acids, preferably at least 10 amino acids, more preferably at least 20 amino acids, most preferably at least 50 amino acids, located C-terminal of the fifth transmembrane region and N-terminal of the sixth transmembrane region of the Icrac-Mlsnl protein. Preferred is a consecutive sequence taken from the amino acid sequence of SEQ ID N°14, 15, 16 or 17. More preferred are the sequences of SEQ ID N°14, 15, 16 or 17.
A further object of the invention is purified or isolated human Icrac-Mlsnl extra-cellular loop-3 region, wherein the extra-cellular loop-3 region has at least 90%, preferably 95%, more preferably 98% and most preferably 99% amino acid identity with any of the peptides of amino acid sequences SEQ ID N°14, 15, 16 or 17, or fragments thereof.

The inventors believe that peptides which contain both intra- and extra- cellular loop regions and transmembrane regions but lacking associated N-and C-terminal regions could play a role in the understanding of Icrac and the development of modulators of Icrac and diagnostic tools. Therefore, an eighth object of the invention is purified or isolated human Icrac-Mlsnl peptide containing both intra- and extra-cellular loop regions, wherein said intra-and extra-cellular loop region comprises a consecutive sequence of at least 50 amino acids, preferably at least 100 amino acids and most preferably at least 250 amino acids, located C-terminal to the N-terminal region and N-terminal to the C-terminal region of the Icrac-Mlsnl protein. Preferred is a consecutive sequence taken from the amino acid sequence of SEQ ID N°18 or 19. More preferred is the sequence of SEQ ID N°18 or 19.
A further object of the invention is purified or isolated human Icrac-Mlsnl peptide containing both intra-and extra-cellular loop regions, wherein the intra- and extra-cellular loop region has at least 90%, preferably 95%, more preferably 98% and most preferably 99% amino acid identity with any of the peptides of amino acid sequences SEQ ID N°18 or 19, or fragments thereof.

Amino acid identity may be determined using various methods known in the art. In particular, amino acid identity (or sequence homology) may be assessed by commercially available computer programs, such as CLUSTAL or BLAST (NCBI). Various search or alignment parameters may also be used. In a preferred embodiment, % amino acid (or nucleic acid) identity is determined using the CLUSTAL-W program (Compugen).

### Nucleic acids encoding peptides of Icrac-Mlsnl

This invention also relates generally to the use of nucleic acid sequences encoding said Icrac-Mlsnl peptides for the development of screening and diagnostic technologies.

An object of the invention is a purified or isolated nucleic acid sequence encoding the human Icrac-Mlsnl N-terminal region, wherein the N-terminal region is encoded by a consecutive sequence of at least 75 nucleotides, preferably at least 300 nucleotides, more preferably at least 750 nucleotides and most preferably at least 1500 nucleotides, located 5' with respect to nucleotides encoding the first transmembrane region of the Icrac-Mlsnl protein. Preferred is a consecutive sequence taken from the nucleic acid sequences of SEQ ID N°24 or 25. More preferred is the sequence of SEQ ID N°24 or 25.

A further object of the invention is a purified or isolated nucleic acid sequence encoding the human Icrac-Mlsnl N-terminal region, wherein the nucleic acid has at least 80%, preferably 90%, more preferably 95% and most preferably 98% nucleic acid identity with any of the nucleotide sequences or SEQ ID N°24 or 25, or a complementary sequences thereto.
Another object of the invention is a purified or isolated nucleic acid sequence encoding a polypeptide, wherein said nucleic acid encodes a polypeptide having at least 90%, preferably, 95, more preferably 98% and most preferably 99% with any of the polypeptides of SEQ ID N°2 or 3.

A further object of the invention is a purified or isolated nucleic acid sequence encoding the human Icrac-Mlsnl C-terminal region, wherein the C-terminal region is encoded by a consecutive sequence of at least 75 nucleotides, preferably at least 150 nucleotides, more preferably at least 300 nucleotides and most preferably at least 600 nucleotides, located 5' with respect to nucleotides encoding the sixth transmembrane region of the Icrac-Mlsnl protein. Preferred is a consecutive sequences taken from the nucleic acid sequence of SEQ ID N°26 or 27. More preferred is the sequence of SEQ ID N°26 or 27.
A further object of the invention is a purified or isolated nucleic acid sequence encoding the human Icrac-Mlsnl C-terminal region, wherein the nucleic acid has at least 80%, preferably 90%, more preferably 95% and most preferably 98% nucleic acid identity with any of the nucleotide sequences or SEQ ID N°26 or 27, or a complementary sequences thereto.
A further object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N°4 or 5.

A further object of the invention resides is a purified or isolated nucleic acid sequence encoding the human Icrac-Mlsnl intra-cellular loop-1 region, wherein the intra-cellular loop-1 region is encoded by a consecutive sequence of at least 21 nucleotides, preferably at least 30 nucleotides, more preferably at least 45 nucleotides, located 3' with respect to nucleotides encoding the second transmembrane region and 5' with respect to nucleotides encoding the third transmembrane region of the Icrac-Mlsnl protein. Preferred is a consecutive sequence taken from the nucleic acid sequence of SEQ ID N°28 or 29. More preferred is the sequence of SEQ ID N°28 or 29.
A further object of the invention is a purified or isolated nucleic acid sequence encoding the human Icrac-Mlsnl loop-1 region, wherein the nucleic acid has at least 80%, preferably 90%, more preferably 95% and most preferably 98% nucleic acid identity with any of the nucleotide sequences or SEQ ID N°28 or 29, or a complementary sequences thereto.
Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N°6 or 7.

A further object of the invention is a purified or isolated nucleic acid sequence encoding the human Icrac-Mlsnl intra-cellular loop-2 region, wherein the intra-cellular loops-2 region is encoded by a consecutive sequence of at least 21 nucleotides, preferably at least 30 nucleotides, located 3' with respect to nucleotides encoding the fourth transmembrane region and 5' with respect to nucleotides encoding the fifth transmembrane region of the Icrac-Mlsnl protein. Preferred is a consecutive sequence taken from the nucleic acid sequence of SEQ ID N°30 or 31. More preferred is the sequence of SEQ ID N°30 or 31.
A further object of the invention is a purified or isolated nucleic acid sequence encoding the human Icrac-Mlsnl intra-cellular loop-2 region, wherein the nucleic acid has at least 80%, preferably 90%, more preferably 95% and most preferably 98% nucleic acid identity with any of the nucleotide sequences or SEQ ID N°30 or 31, or a complementary sequences thereto.
Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N°8 or 9.

A further object of the invention is a purified or isolated nucleic acid sequence encoding the human Icrac-Mlsnl extra-cellular loop-1 region, wherein the extra-cellular loops-1 region is encoded by a consecutive sequence of at least 15 nucleotides, located 3' with respect to nucleotides encoding the first transmembrane region and 5' with respect to nucleotides encoding the second transmembrane region of the Icrac-Mlsnl protein. Preferred is a consecutive sequence taken from the nucleic acid sequence of SEQ ID N°32 or 33. More preferred is the sequence of SEQ ID N°32 or 33.
A further object of the invention is purified or isolated nucleic acid sequence encoding the human Icrac-Mlsnl extra-cellular loop-1 region, wherein the nucleic acid has at least 80%, preferably 90%, more preferably 95% and most preferably 98% nucleic acid identity with any of the nucleotide sequences or SEQ ID N°32 or 33, or a complementary sequences thereto.
Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N°10 or 11.

A further object of the invention is purified or isolated nucleic acid sequence encoding the human Icrac-Mlsnl extra-cellular loop-2 region, wherein the extra-cellular loops-2 region is encoded by a consecutive sequence of at least 15 nucleotides, located 3' with respect to nucleotides encoding the third transmembrane region and 5' with respect to nucleotides encoding the fourth transmembrane region of the Icrac-Mlsnl protein. Preferred is a consecutive sequence taken from the nucleic acid sequence of SEQ ID N°34 or 35. More preferred is the sequence of SEQ ID N°34 or 35.
A further object of the invention is purified or isolated nucleic acid sequence encoding the human Icrac-Mlsnl extra-cellular loop-2 region, wherein the nucleic acid has at least 80%, preferably 90%, more preferably 95% and most preferably 98% nucleic acid identity with any of the nucleotide sequences or SEQ ID N°34 or 35, or a complementary sequences thereto.
Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N° 12 or 13.

A further object of the invention is purified or isolated nucleic acid sequence encoding the human Icrac-Mlsnl extra-cellular loop-3 region, wherein the extra-cellular loops-3 region is encoded by a consecutive sequence of at least 21 nucleotides, preferably at least 30 nucleotides, more preferably at least 60 nucleotides and most preferably at least 150 nucleotides 3' with respect to nucleotides encoding the fifth transmembrane region and 5' with respect to nucleotides encoding the sixth transmembrane region of the Icrac-Mlsnl protein. Preferred is a consecutive sequence taken from the nucleic acid sequence of SEQ ID N°36, 37 or 38. More preferred is the sequence of SEQ ID N°36, 37 or 38.
A further object is purified or isolated nucleic acid sequence encoding the human Icrac-Mlsnl extra-cellular loop-3 region, wherein the nucleic acid has at least 80%, preferably 90%, more preferably 95% and most preferably 98% nucleic acid identity with any of the nucleotide sequences or SEQ ID N°36, 37 or 38, or a complementary sequences thereto.
Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N° 14, 15, 16 or 17.

An other object of the invention is purified or isolated nucleic acid sequence encoding the human Icrac-Mlsnl peptide containing both intra- and extra-cellular loop regions, wherein said intra- and extra-cellular loop region is encoded by a consecutive sequence of at least 150 nucleotides, preferably at least 300 nucleotides and most preferably at least 750 nucleotides 3' with respect to nucleotides encoding the N-terminal region and 5' with respect to nucleotides encoding the C-terminal regions of the Icrac-Mlsnl protein. Preferred is a consecutive sequence taken from the nucleic acid sequence of SEQ ID N°39 or 40. More preferred is the sequence of SEQ ID N°39 or 40.
Another object of the invention is purified or isolated nucleic acid sequence encoding the human Icrac-Mlsnl peptide containing both intra- and extra-cellular loop regions, wherein the nucleic acid has at least 80%, preferably 90%, more preferably 95% and most preferably 98% nucleic acid identity with any of the nucleotide sequences or SEQ ID N°39 or 40, or a complementary sequences thereto.

Another object of the invention is a purified or isolated nucleic acid sequence encoding a polypeptide, wherein said nucleic acid encodes a polypeptide having at least 90%, preferably, 95%, more preferably 98% and most preferably 99% with any of the polypeptides of SEQ ID N°18 or 19.

Nucleic acid sequence identity may be determined using various methods known in the art. In particular, nucleic acid sequence identity (or sequence homology) may be assessed by commercially available computer programs, such as CLUSTAL or BLAST (NCBI). Various search or alignment parameters may also be used. In a preferred embodiment, % amino acid (or nucleic acid) identity is determined using the CLUSTAL-W program (Compugen).

### TRANSCRIPTION PROMOTERS OF Icrac-Mlsnl

The inventors have identified three genomic nucleic acid sequences 5' to the published codon encoding Methionine 1 of the Icrac-Mlsnl gene that encode transcription promoter sequences. The inventors believe that nucleic acids encoding transcription promoter(s) elements of Icrac-Mlsnl are targets for the development of therapeutics which modulate Icrac activity. A first preferred nucleic acid sequence is a nucleic acid sequence which encodes an Icrac-Mlsnl transcription promoter of SEQ ID N°41 or a complementary sequence thereto. A further object of the invention consists of nucleic acid sequences having at least 80%, preferably 90%, more preferably 95% and most preferably 98% nucleotide identity with the nucleotide sequence of SEQ ID N°41 or a complementary sequence thereto.

A second preferred nucleic acid sequence is a nucleic acid sequence which encodes an Icrac-Mlsnl transcription promoter of SEQ ID N°42 or a complementary sequence thereto. A further object of the invention consists of nucleic acid sequences having at least 80%, preferably 90%, more preferably 95% and most preferably 98% nucleotide identity with the nucleotide sequence of SEQ ID N°42 or a complementary sequence thereto.

A third preferred nucleic acid sequence is a nucleic acid sequence which encodes an Icrac-Mlsnl transcription promoter of SEQ ID N°43 or a complementary sequence thereto. A further object of the invention consists of nucleic acid sequence having at least 80%, preferably 90%, more preferably 95% and most preferably 98% nucleotide identity with the nucleotide sequence of SEQ ID N°43 or a complementary sequence thereto.

### EXPRESSION OF Icrac-Mlsnl

The inventors discovered that the Icrac-Mlsnl gene was expressed in U937 cells differentiated into a macrophage phenotype, and that the product of this gene functions as a calcium channel, more specifically as an Icrac channel. Mlsnl-specific primers were designed and used to screen for Icrac-Mlsnl gene expression in various cells. The composition of these primers are indicated in SEQ ID N°44 to 47. With said primer pairs, Icrac-Mlsnl-specific PCR products were obtained from U937 cells. Furthermore, cDNA subtraction experiments were performed using DNA preparations from differentiated and undifferentiated U937 cells. The results obtained demonstrate that the expression of the Icrac-Mlsnl gene product (TrpC8) in U937 cells is differentially increased in dbcAMP-differentiated U937 cells (macrophage-like) as compared to undifferentiated U937-cells (monocyte like). The upregulation of the Icrac-Mlsnl gene correlates with the expression of the Icrac phenotype in U937 cells (18) and thus establishes that expression upregulation of Icrac-Mlsnl in these cells coincides with the increased Icrac phenotype. Sequence specific evidence for Icrac-Mlsnl involvement in the Icrac phenotype was obtained by the demonstration that Icrac-Mlsnl-specific anti-sense treatment of U937 cells during dbcAMP induced differentiation could significantly decrease the calcium signal in these cells.

Accordingly, this invention unexpectedly demonstrates that Icrac-Mlsnl is expressed in immune cells such as macrophages and that the down-regulation of the Icrac-Mlsnl gene expression during differentiation of U937 cells to macrophage like cells decreases calcium influx in these cells. This invention also demonstrates that Icrac-Mlsnl gene product expression is up-regulated during U937 cell differentiation to macrophage like cells, which correlates with the maturation stage of these cells and their ability to exhibit the Icrac phenotype

### PRODUCTION OF Icrac-Mlsnl POLYPEPTIDES OR NUCLEIC ACIDS

### Recombinant expression vecotors

The present invention also encompasses a family of recombinant vectors comprising any one of the nucleic acids described herein. Thus, the invention further deals with a recombinant vector comprising a nucleic acid selected from the group consisting of:
(a) a purified or isolated nucleic acid encoding Icrac-Mlsnl, preferably human Icrac-Mlsnl or Icrac-Mlsnl transcription promoter element(s), and more preferably a polypeptide having at least 90% amino acid identity with a polypeptide selected from the group consisting of; nucleic acids encoding Icrac-Mlsnl, such as SEQ ID N°1, nucleic acids encoding the N-terminal region of Icrac-Mlsnl, such as SEQ ID N°2 and 3, nucleic acids encoding C-terminal region of Icrac-Mlsnl, such as SEQ ID N°4 and 5, nucleic acids encoding Intra-cellular loops of Icrac-Mlsnl, such as SEQ ID N°6 to 9, nucleic acids encoding extra-cellular loops 1 and 2 of Icrac-Mlsnl, such as SEQ ID N°10 to 13, nucleic acids encoding extra-cellular loop 3 of Icrac-Mlsnl, such as SEQ ID N°14 to 17, nucleic acids encoding both intra- and extra cellular loops of Icrac-Mlsnl, such as SEQ ID N°18 and 19 and nucleic acids encoding transcription promoter elements of SEQ ID N°41 to 43, or a sequence complementary thereto;
(b) a purified or isolated nucleic acid having at least 80% nucleotide identity with a polynucleotide selected from the group consisting of the nucleotide sequences of SEQ ID N°23 to43, or a sequence complementary thereto;
(c) a purified or isolated polynucleotide comprising at least 10 consecutive nucleotides of a nucleic acid described in (a) or (b), or a sequence complementary thereto.
In a first preferred embodiment a recombinant vector of the invention is used to amplify the inserted polynucleotide derived from the nucleic acid encoding a Icrac-Mlsnl of the invention or Icrac-Mlsnl transcription promoter in a suitable host cell, this polynucleotide being amplified every time the recombinant vector replicates.
A second preferred embodiment of the recombinant vectors according to the invention consists of expression vectors comprising a nucleic acid encoding an Icrac-Mlsnl of the invention, preferably a nucleic acid encoding a human Icrac-Mlsnl, and more preferably a nucleic acid encoding a polypeptide selected from the group consisting of the amino acid sequences of SEQ ID N°1 to 22.
Recombinant expression vectors comprising a nucleic acid encoding the peptide fragments of an Icrac-Mlsnl that are specified in the present specification are also part of the invention.
Within certain embodiments, expression vectors can be employed to express Icrac-Mlsnl of the invention or a peptide fragment thereof which can then be purified and for example, be used as a immunogen in order to raise specific antibodies directed against said Icrac-Mlsnl protein or a peptide fragment thereof.
In another embodiment, the expression vectors are used for constructing transgenic animals and also for gene therapy, notably for anti-sense therapy.

Expression requires that appropriate signals are provided in the vectors, said signals including various regulatory elements such as enhancers/promoters from both viral and mammalian sources that drive expression of the genes of interest in host cells. The regulatory sequences of the expression vectors of the invention are operably linked to the nucleic acid encoding the Icrac-Mlsnl protein of interest or a peptide fragment thereof.
As used herein, the term " operably linked " refers to a linkage of polynucleotide elements in a functional relationship. For instance, a promoter or an enhancer is operably linked to a coding sequence if it affects the transcription of the coding sequence.
More precisely, two DNA molecules (such as a polynucleotide containing a promoter region and a polynucleotide encoding a desired polypeptide or polynucleotide) are said to be " operably linked" if the nature of the linkage between the two polynucleotides does not : (1) result in the introduction of a frame-shift mutation or (2) interfere with the ability of the polynucleotide containing the promoter to direct the transcription of the coding polynucleotide.
Generally, recombinant expression vectors will include origins of replication, selectable markers, permitting transformation of the host cell, and a promoter derived from a highly expressed gene to direct transcription of a downstream structural sequence. The heterologous structural sequence is assembled in an appropriate frame with the translation, initiation and termination sequences, and preferably a leader sequence capable of directing sequences of the translated protein into the periplasmic space or the extra-cellular medium.
In a specific embodiment wherein the vector is adapted for transfecting and expressing desired sequences in mammalian host cells, preferred vectors will comprise an origin of replication from the desired host, a suitable promoter and an enhancer, and also any necessary ribosome binding sites, polyadenylation site, transcriptional termination sequences, and optionally 5'-flanking non-transcribed sequences.
DNA sequences derived from the SV 40 viral genome, for example SV 40 origin, early promoter, enhancer, and polyadenylation sites may be used to provide the required non-transcribed genetic elements.

Additionally, a recombinant expression vector of the invention advantageously also comprises an untranscribed polynucleotide located at the 3' end of the coding sequence (ORF), this 3'-UTR polynucleotide being useful for stabilizing the corresponding mRNA or for increasing the expression rate of the vector insert if this 3'-UTR harbors regulation signal elements such as enhancer sequences.

Suitable promoter regions used in the expression vectors according to the invention are chosen taking into account the host cell in which the heterologous nucleic acids have to be expressed.
A suitable promoter may be heterologous with respect to the nucleic acid for which it controls the expression, or alternatively can be endogenous to the native polynucleotide containing the coding sequence to be expressed.
Additionally, the promoter is generally heterologous with respect to the recombinant vector sequences within which the construct promoter/coding sequence has been inserted.
Preferred bacterial promoters are the LacI, LacZ, T3 or T7 bacteriophage RNA polymerase promoters, the lambda PR, PL and trp promoters (a EP-0 036 776), the polyhedrin promoter, or the p10 protein promoter from *baculovirus* (kit Novagen; Smith et al., (1983); O'Reilly et al. (1992).

Preferred selectable marker genes contained in the expression recombinant vectors of the invention for selection of transformed host cells are preferably dehydrofolate reductase or neomycin resistance for eukaryotic cell culture, TRP1 for *S. cerevisiae* or tetracycline, rifampicin or ampicillin resistance in *E*. *coli,* or Levamsaccharase for *Mycobacteria,* this latter marker being a negative selection marker.

Preferred bacterial vectors of the invention are listed hereafter as illustrative but not limitative examples:
pQE70, pQE60, pQE-9 (Quiagen), pD10, phagescript, psiX174, p.Bluescript SK, pNH8A, pNH16A, pNH18A, pNH46A (Stratagene); pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIA express).
Preferred bacteriophage recombinant vectors of the invention are P1 bacteriophage vectors such as described by Sternberg N.L. (1992;1994).
A suitable vector for the expression of Icrac-Mlsnl polypeptide of the invention or a fragment thereof, is a baculovirus vector that can be propagated in insect cells and in insect cell-lines. A specific suitable host vector system is the pVL 1392/1393 *baculovirus* transfer vector (Pharmingen) that is used to transfect the SF9 cell line (ATCC N°CRL 1711) which is derived from *spodoptera frugiperda*.

The recombinant expression vectors from the invention may also be derived from an adenovirus such as those described by Feldman and Steig. (1996) or Ohno et al. (1994).
Another preferred recombinant adenovirus according to this specific embodiment of the present invention is the human adenovirus type two or five (Ad 2 or Ad 5) or an adenovirus of animal origin (French Patent Application n°FR 93 05 954).
Particularly preferred retrovirus as for the preparation or construction of retroviral *in vitro* or *in vivo* gene delivery vehicles of the present invention include retroviruses selected from the group consisting of Mink-Cell Focus Inducing Virus, murine sarcoma virus, and Ross Sarcoma Virus. Other preferred retroviral vectors are those described in Roth et al. (1996), in PCT Application WO 93/25 234, in PCT Application WO 94/06920, and also in Roux et al. (1989), Julan et al.(1992) and Nada et al. (1991).
Yet, another viral vector system that is contemplated by the invention consist in the adeno associated viruses (AAV) such as those described by Flotte et al. (1992), Samulski et al. (1989) and McLaughlin et al. (1996).

Thus, a further object of the invention consists of a recombinant expression vector comprising a nucleic acid encoding an Icrac-Mlsnl or a peptide fragment thereof or a variant thereof, wherein said nucleic acid is operably linked to a promoter sequence.

In a preferred embodiment, this nucleic acid encodes a human Icrac-Mlsnl, a fragment of human Icrac-Mlsnl, or Icrac-Mlsnl transcription promoter, and preferably that of SEQ ID N°23 and nucleic acid sequence encoding peptide sequence of SEQ ID N°1, or a variant or a peptide fragment thereof. Preferred fragments include those of SEQ ID N° 2-22 and 24 to 40. Preferred Icrac-Mlsnl transcription promoters include those of SEQ ID N°41 to 43.
In a specific embodiment of this invention, any of the identified Icrac-Mlsnl promoter elements of SEQ ID N°41 to 43 could be used to modulate the expression of a reporter molecule, as part of an expression construct. Furthermore, these Icrac-Mlsnl transcription promoter elements could be used to regulate Icrac-Mlsnl gene of gene fragment expression as part of a construct.

### Host cells expressing Icrac-Mlsnl

Host cells that endogenously express Icrac-Mlsnl or have been transformed or transfected with one of the nucleic acids described herein, or with one of the recombinant vector, particularly recombinant expression vector, described herein are also part of the present invention.
Also included are host cells that are transformed (prokaryotic cells) or are transfected (eukaryotic cells) with a recombinant vector such as one of those described above. Preferred host cells used as recipients for the expression vectors of the invention are the following:
(a) prokaryotic host cells: *Escherichia coli,* strains. (i.e. DH5-α, strain) *Bacillus subtilis, Salmonella typhimurium* and strains from species like *Pseudomonas, Streptomyces* and *Staphylococcus;*
(b) eukaryotic host cells: T-cell lines (ECACC U937, 85011440; ECACC J.CaM1.6, 96060401 ; ECACC Jurkat E6.1, 88042803 and ECACC J45.01, 93031145), HeLa cells (ATCC N°CCL2; N°CCL2.1; N°CCL2.2), Cv 1 cells (ATCC N°CCL70), COS cells (ATCC N°CRL 1650; N°CRL 1651), Sf-9 cells (ATCC N°CRL 1711), C127 cells (ATCC N°CRL-1804), 3T3 cells (ATCC N°CRL-6361), CHO cells (ATCC N°CCL-61), human kidney 293 cells (ATCC N° 45504; N°CRL-1573), BHK (ECACC N°84100 501; N°84111301), PC12 (ATCC N° CRL-1721), NT2, SHSY5Y (ATCC N° CRL-2266), NG108 (ECACC N°88112302) and F11, SK-N-SH (ATCC N° CRL-HTB-11), SK-N-BE(2) (ATCC N° CRL-2271), IMR-32 (ATCC N° CCL-127). A preferred system to which the gene of the invention can be expressed are cell lines such as T-cell lines, B-cell lines, mast cell lines, jurkat cell lines, U937 cell lines, KU-812 cell lines, COS cells, 3T3 cells, HeLa cells, 292 cells and CHO cells. A most preferred system for the efficient expression of Icrac-Mlsnl involves the use of T-cell lines. The gene can be expressed through an endogenous promoter of native T-cells, or through an exogenous promoter. Suitable exogenous promoters include such as SV40 and CMV, or perhaps a eukaryotic promoter such as the tetracycline promoter. The preferred promoter when Icrac-Mlsnl is endogenously expressed is an endogenous promoter. A prefered promoter in a recombinant cell line would be CMV.

In a specific embodiment of the host cells described above, these host cells have also been transfected or transformed with a polynucleotide or a recombinant vector allowing the expression of a natural ligand of Icrac-Mlsnl or a modulator of Icrac-Mlsnl.

The present invention also concerns a method for producing one of the Icrac-Mlsnl polypeptides or peptides described herein and especially a polypeptide selected from the group consisting the amino acid sequences of SEQ ID N°1 to 22, wherein said method comprises the steps of:
(a) inserting the nucleic acid encoding the desired Icrac-Mlsnl polypeptide or peptide fragment thereof in an appropriate vector;
(b) culturing, in an appropriate culture medium, a host cell previously transformed or transfected with the recombinant vector of step (a);
(c) harvesting the culture medium thus conditioned or lyse the host cell, for example by sonication or by an osmotic shock;
(d) separating or purifying, from said culture medium, or from the pellet of the resultant host cell lysate, the thus produced Icrac-Mlsnl polypeptide of interest.
   In a first preferred embodiment of the above method, the nucleic acid to be inserted in the appropriate vector has previously undergone an amplification reaction, using a pair of primers.

Preferred primers used for such an amplification reaction are primer pairs which amplify nucleic acids encoding the Icrac-Mlsnl open reading frame and fragments thereof.

In a second preferred embodiment of the above method, the polypeptide thus produced is further characterized, for example by binding onto an immuno-affinity chromatography column on which polyclonal or monoclonal antibodies directed to Icrac-Mlsnl polypeptide or a peptide fragment thereof, have previously been immobilised.

Purification of the recombinant Icrac-Mlsnl proteins according to the present invention or a peptide fragment thereof may be carried out by passage onto a nickel or copper affinity chromatography column.

In another embodiment, the Icrac-Mlsnl polypeptides or peptide fragments thus obtained may be purified, for example, by high performance liquid chromatography, such as reverse phase and/or cationic exchange HPLC, as described by Rougeot et al. (1994).

The reason to prefer this kind of peptide or protein purification is the lack of by-products formed in the elution samples which renders the resultant purified protein or peptide more suitable for therapeutic use.

### Production of Icrac-Mlsnl or a fragment thereof

The Icrac-Mlsnl protein or fragments thereof can be prepared using recombinant technology, cell lines or chemical synthesis. Recombinant technology and chemical synthesis of the Icrac-Mlsnl or fragments thereof can allow the modification of the gene encoding the Icrac-Mlsnl to include such features as recognition tags, cleavage sites and modifications of the Icrac-Mlsnl polypeptide or fragments thereof. For efficient polypeptide production, the endogenous expression system or recombinant expression system should allow the Icrac-Mlsnl polypeptide to be expressed at and transported to the cell surface in a functional form or allow production of Icrac-Mlsnl fragments which can be purified. Preferred cell lines are those which allow high levels of expression of Icrac-Mlsnl or fragments thereof. Such cell lines include cell lines which naturally expresss Icrac-Mlsnl or common mammalian cell lines expressing Icrac-Mlsnl such as CHO cells and COS cells, etc., or more specific immune cell lines such as T-cell lines. However, other cell types which are commonly used for recombinant protein production such as insect cells, amphibian cells such as oocytes, yeast and procaryotic cell lines such as *E.coli* can also be considered.

The Icrac-Mlsnl or fragments thereof can be utilised in a ligand screen either as a purified protein, as a protein chimera such as those described in phage display, as a cell membrane (lipid or detergent) preparation, or in intact cells.
The Icrac-Mlsnl polypeptide or fragment thereof can be utilised in a functional screen format or ligand binding screen format. Examples of both screening formats are provided below.

### SCREENING FOR Icrac-Mlsnl LIGANDS

The present invention also concerns methods for screening ligand substances or molecules that are able to modulate the biological activity of the Icrac-Mlsnl gene or gene product of the invention. For example diseases where there is a down-regulation of the immune system and conditions associated with virus and micro-organism infection, enhancement of the activity of Icrac-Mlsnl would be beneficial. In conditions such as asthma, inflammation and auto-immune disease it would be beneficial to identify ligands which can down-regulate the activity of the Icrac-Mlsnl gene.

In this regard, this invention now discloses the use of a Icrac-Mlsnl gene product for the screening of compound(s) that modulate the activity of immune cells, in particular antigen presenting cells, more specifically for the screening of compound(s) that modulate the activity of macrophages, *in vitro, ex vivo* or *in vivo.*

This invention also discloses the use of Icrac-Mlsnl promoter elements for the screening of compounds that modulate the activity of immune cells, in particular antigen presenting cells, more specifically for the screening of compounds that modulate the activity of macrophages, *in vitro, ex vivo or in vivo.*

Several screening assays can be used within the instant invention, such as binding assays or functional assays. In a particular embodiment, this invention more specifically relates to methods of screening for immuno-modulatory compounds, comprising;
a) contacting a test compound with Icrac-Mlsnl gene, an Icrac-Mlsnl gene product or Icrac-Mlsnl promoter element and,
b) measuring the ability of said test compound to interact with the Icrac-Mlsnl gene, Icrac-Mlsnl gene product or transcription promoter element (i.e., binding assay).

In another embodiment, the screening method comprises;
a) contacting a test compound with an Icrac-Mlsnl expressing cell, more particularly an antigen presenting cell (or a macrophage) and,
b) determining the ability of said test compound to affect Icrac-Mlsnl gene or an Icrac-Mlsnl gene product activity within the cells.

In a further embodiment, the screening method comprises contacting a test compound with a cell expressing a reporter gene under the control of Icrac-Mlsnl transcription promoter elements, more particularly an antigen presenting cell (or a macrophage), expressing the said promoter/reporter construct and determining the ability of the said test compound to modulate reporter expression.

Ligand binding screens to the Icrac-Mlsnl gene, polypeptide, a fragment thereof or Icrac-Mlsnl transcription promoter element can allow rapid identification of ligands which are capable of interacting with the gene or product(s). Further testing may be required to determine the therapeutic activity of these ligands. Development of a functional screen, whereby the activity of Icrac-Mlsnl is measured (or reporter activity measured) upon ligand binding allows ligands to be rapidly identified which modulate the activity of Icrac-Mlsnl.

This invention also relates to the use of the Icrac-Mlsnl gene product to screen for any molecule involved in the Icrac-Mlsnl gene pathway, i.e. for any protein or peptide that interact with Icrac-Mlsnl. Such targets (or Icrac-Mlsnl partners or signaling molecules) can be screened by protein-protein interaction analysis techniques (double-hybrid system for instance), or any other method known to the skilled artisan.

### Ligand binding screening method (phage display, flashplate)

Ligand binding screening comprises, preferably, contacting a test compound with an Icrac-Mlsnl gene product or Icrac-Mlsnl transcription promoter element(s) and determining the ability of said test compound to interact with the Icrac-Mlsnl gene product or transcription promoter element(s). In a typical embodiment, the ligand binding screen comprises contacting one or several (in parallel) test compounds with a Icrac-Mlsnl polypeptide (e.g., protein or a fragment thereof) or transcription promoter element(s), in the presence (or absence, as a reference) of a Icrac-Mlsnl ligand or transcription promoter ligand, and assessing the ability of the test compound(s) to bind Icrac-Mlsnl transcription polypeptide or promoter element(s) by assessing ligand binding.

The Icrac-Mlsnl polypeptide can be part of an intact cell, membrane preparation or a purified polypeptide, optionally attached to a support, such as beads, column plate, etc. The polypeptide is preferably characterized by comprising an amino acid sequence selected from SEQ ID N°1 to 22.
The Icrac-Mlsnl transcription promoter element(s) can preferably but not exclusively be a purified nucleic acid, optionally attatched to a support such as beads, column, plate etc.. The nucleic acid encoding the promoter elements is preferably characterized by comprising nucleic acid sequences selected from SEQ ID N°41, 42 or 43.
The test compound can be a peptide or a protein or an antibody or chemical entity, either alone or in combination(s) or in a mixture with any other substance. The test compound may even represent a library of compounds. Optionally, excess non Icrac-Mlsnl bound ligand can be removed by separation. Separation can take the form of washing /filtering or centrifugation (to pellet the Icrac-Mlsnl protein). In this latter case, the supernatant can then be removed and the Icrac-Mlsnl re-suspended in buffer.

The binding is preferably performed in the presence of a Icrac-Mlsnl ligand or transcription promoter element ligand, to allow an assessment of the binding activity of each test compound. In particular, in a preferred embodiment, a labeled Icrac-Mlsnl ligand or promoter element ligand is used and the binding activity of the test compound is determined by assessing any modulation of the ligand binding.

The ligand may be contacted with the Icrac-Mlsnl polypeptide or transcription promoter element either before, simultaneously or after the test compound.

The ligand should be detectable and/or quantifiable. To achieve this, the ligand can be labeled in a number of ways, such as with a chromophore, radioactive, fluorescent, phosphorescent, enzymatic or antibody label, for instance. If the ligand is not directly detectable it must be amenable to detection and quantification by secondary detection, which may employ the above technologies. Preferably (in the case of certain detection methods), unbound ligand is removed from the mixture prior to detecting ligand binding, as described above.
Alternatively the Icrac-Mlsnl polypeptide or fragment thereof of Icrac-Mlsnl transcription promoter element(s) can be detectable or quantifiable. This can be achieved in a similar manner to that described above.

Preferred examples of such Icrac-Mlsnl ligands that can be used in the instant invention include any product that is known to interact with Icrac-Mlsnl, such as an antibody, physiological ligand or receptor, or synthetic ligands or Icrac-Mlsnl inhibitors. Specific examples of such ligands include pyrazole compounds as described in WO99/19303, which exhibit calcium release-dependent calcium channel inhibitory effect. These compounds may be labeled according to various techniques, including radioactivity or fluorescence. More specific compounds are compounds SEW04225, KM02940, KM03000 and GK02421 as listed in the Maybridge Co. Trial Drug Catalogue (UK, Cornwall, published August 95).
Preferred examples of Icrac-Mlsnl transcription promoter element ligands that can be used in this instant invention include any product that is known to interact with Icrac-Mlsnl promoter elements, such as polymerases, antibodies, other naturally occuring ligands or synthetic ligands.

Binding of the test compound modifies the interaction of the ligand with the binding site and changes the affinity or binding of ligand for/to its binding site. The difference between the observed amount of ligand bound relative to the theoretical maximum amount of ligand bound (or to the ligand bound in the absence of a test compound under the same conditions) is a reflection of the binding ability (and optionally the amount and/or affinity) of a test compound to bind Icrac-Mlsnl or its transcription promoter elements.

Alternatively, the amount of test compound bound to the Icrac-Mlsnl protein or a fragment thereof, or transcription promoter element(s) can be determined by a combination of chromatography and mass spectroscopy. The amount of test compound bound to the Icrac-Mlsnl protein or a fragment thereof or transcription promoter element(s) can also be determined by direct measurement of the change in mass upon compound or ligand binding to Icrac-Mlsnl or transcription promoter element(s). This can be achieved with technologies such as Biacore (Amersham Pharmacia). Alternatively, the Icrac-Mlsnl protein or a fragment thereof, or promoter element(s), the compound or the ligand can be fluorescently labeled and association of Icrac-Mlsnl protein or promoter element(s) with the compound can be followed by changes in Fluorescence Energy Transfer (FRET).

### Functional screening Method

In addition to, or in replacement of the binding screen, functional screens may be used to identify or characterize immuno-modulators or Icrac-Mlsnl modulators. Such a functional screen is advantageously transferable to high throughput, to allow the screening of large numbers of test compounds. The functional screen essentially comprises contacting a Icrac-Mlsnl-expressing cell with a test compound, and assessing the ability of said test compound to affect Icrac-Mlsnl gene pathway or Icrac-Mlsnl gene product activity within the cells. This can be performed by measuring calcium fluxes, activation potential (or profile) or signal transduction pathway (e.g., selected cellular gene expression level) within said cells.

In a particular embodiment, the present invention thus resides in a method of screening (or identifying or selecting or characterizing) immuno-modulators, comprising (i) exposing a Icrac-Mlsnl-expressing cell to one or several test compounds and (ii) detecting the compound that induce a modulation of the calcium flux or the activation potential in said cells. More preferably, the cells are recombinant cells expressing a Icrac-Mlsnl polypeptide, or immune cells, in particular antigen-presenting cells, more preferably macrophages.

In a first preferred variant of this embodiment, the functional screening method comprises the following steps:
(1) obtaining a recombinant cell expressing Icrac-Mlsnl gene or gene product,
(2) exposing said recombinant cell to a substance or molecule to be tested; and
(3) measuring the change in calcium flux or activation potential within the exposed recombinant cell.

In a preferred embodiment, the cell express a functional Icrac-Mlsnl polypeptide comprising all or part of SEQ ID N°1, in particular a polypeptide comprising a sequence selected from SEQ ID N° 1 to 22.

### Calcium Flux

Calcium Flux may be measured according to various techniques, such as for instance using sodium sensitive dyes such as Fura II. In a particular embodiment, compounds are selected for their ability to cause an increase in calcium concentration within the cell. In an other embodiment, compounds are selected for their ability to cause a decrease in calcium concentration within the cell. Alternatively, compounds are selected for their ability to alter the rate of recovery of the calcium channel.

### Electrophysiology

In another embodiment of the functional screening method, substances or molecules of interest are selected for their ability to induce changes in the activation potential, the inactivation time, or the rate of recovery of the sodium channel. Preferred molecules or substances are those inducing a decrease in the inactivation potential, and/or a decrease in the rate of inactivation, and/or which decreases the rate of recovery from inactivation, as compared with the same measures performed in the absence of the substance of molecule to be tested.

In an other preferred variant, the functional screen is based on a measure of selected gene expression or activity within the cells. In this regard, the present invention now proposes, for the first time, to select Icrac modulators based on their ability to modify the expression pattern of particular genes. More preferably, the selected genes are selected from cytokine genes (in particular interleukin-2, interleukin-8, etc.) and chemokine genes (in particular MIPa). Accordingly, a preferred screening method of this invention comprises contacting Icrac-Mlsnl-expressing cells with one or several test compounds and identifying the compounds that cause a modification in expression or activity of a gene selected from a cytokine or a chemokine gene, within said exposed cells.

Modification in gene expression or activity may be determined by assessing the presence or (relative) level of the gene product in the cell, in particular of the mRNA or polypeptide. Polypeptides may be detected or dosed using affinity reagents, such as antibodies (or fragments or derivatives thereof), either within the cells or at the cell surface, or in the cell culture medium (or supernatant). mRNA levels may be evaluated using conventional techniques (primers, probes, etc). In a specific embodiment, the exposed cells (or any preparation derived therefrom such as membranes, pellets, RNAs, etc) are contacted with a support to which particular affinity reagents are attached (antibodies or nucleic acids).

### Transcription promoter-reporter

In a further embodiment, a promoter-reporter assay can be used to screen for compounds that modulate Icrac-Mlsnl expression. A reporter gene encoding a reporter molecule (i.e. a molecule(s) that can be directly or indirectly detected) is placed 3' with respect to the nucleic acids encoding Icrac-Mlsnl transcription promoter element(s). The position and orientation of which is such that reporter gene expression is initiated from the Icrac-Mlsnl transcription promoter. Preferable transcription promoter elements are chosen from nucleic acids encoded by SEQ ID N°41, 42 or 43. Preferred reporter genes could be those that encode functional β-Galactosidase, Green fluorescence Protein or Luciferase. However, many other reporter molecules could be used, the identity and characteristics of which are known to those skilled in the art.
Further optimization of reporter expression could be achieved with the inclusion of additional nucleic acid elements, as described previously.

The action of test compounds can be measured by exposure of these compounds to cell lines expressing the promoter-reporter construct. Compounds which down regulate the action of the Icrac-Mlsnl promoter elements will manifest by reducing the amount or activity of the reporter in the cell relative to cells not compound treated cells. Compounds which up regulate the action of the Icrac-Mlsnl promoter elements will manifest by increasing the amount or activity of the reporter in the cell relative to cells not compound treated cells.
Compounds which modulate the amount of reporter or activity by other means could be distinguished from those that do not by secondary screening with similar cell lines expressing reporter with non Icrac-Mlsnl transcription promoter elements. Compounds which modulate the reporter in the first screen and not in the second screen could be considered to mediate its action either directly or indirectly through the Icrac-Mlsnl transcription promoter elements.
An assay would comprise the following steps;
a) obtaining a recombinant cell expressing a reporter molecule under the control of an Icrac-Mlsnl transcription promoter Icrac-Mlsnl gene,
b) exposing said recombinant cell to a substance or molecule to be tested ; and Measuring the change in reporter expression or activity in said recombinant cell.

### DIAGNOSTICS ASSAYS AND KITS

The inventors believe that the ability to detect and measure the amount of Icrac-Mlsnl in immune cells would be a valuable tool for the prognosis of aberrant Icrac-Mlsnl and Icrac conditions in humans. The inventors believe that detection of Icrac-Mlsnl in immune cells could be achieved by measuring the amount of expressed Icrac-Mlsnl nucleic acid in immune cells. This could be achieved using a multi-step process:
(a) obtaining a (small) blood sample from a patient (of known volume),
(b) optionally separating immune cells from other blood components. This could be achieved by centrifugation and/or affinity chromatography.
(c) reverse transcribe cellular RNA to cDNA using protocols similar to those described in examples,
(d) amplify Icrac-Mlsnl nucleic acid obtained in step c) by PCR with a plurality of Icrac-Mlsnl specific oligonucleotides capable of hybridizing, under stringent conditions to Icrac-Mlsnl nucleic acids. Such oligonucleotides and amplification conditions have been outlined in examples,
(e) characterize the PCR product. One way to identify the presence of an Icrac-Mlsnl specific PCR product would be to electrophorese an aliquote of amplified nucleic acid in agarose. Such technology has been outlined in examples.

This test could be used to determine if Icrac-Mlsnl is being expressed in immune cells, therefore providing a valuable indication in the prognosis of disorders associated with aberrant Icrac function. Furthemore, an ability to accurately quantitate the level of expression if Icrac-Mlsnl in immune cells would help in the prognosis of the severity of an Icrac associated disorder. Quantitation could be achieved with the inclusion of further step(s). This step(s) would require the assay of a known quantity of nucleic acid which encodes Icrac-Mlsnl.

Co-electrophoresis of samples which have not been subjected to blood products but have been "spiked" with known concentrations of nucleic acids encoding human Icrac-Mlsnl such as SEQ ID N°23 or fragments thereof, or complementary sequences thereto with the test sample, would allow identification and quantitation of levels of Icrac-Mlsnl in the blood sample.

Other aspects and advantages of the present invention will be disclosed in the following experimental section, which should be regarded as illustrative and not limiting the scope of protection.

### EXAMPLES

### 1. A TrpC8 (Mlsnl) gene product is expressed in human U937 cells

cDNA libraries were prepared from U937 cells (these cells being refered to as undifferentiated cells) and dbcAMP differentiated U937 cells (48 hours) using the Clontech SMART system (K1052-1). In brief, U937 cell were cultured in suspension in cell culture media (RPMI 1640 plus 10% heat inactivated FBS) at 37°C in 5 % CO₂, in the presence and absence of differentiating agent (dbcAMP Conc 1µM) (48 hours). Differentiated and undifferentiated cells were harvested by centrifugation at 1,200g for 10 minutes, the supernatent removed from the cell pellet and cellular RNA extracted using FastTrack 2.0 kit (Invitrogen). A SMART library was prepared from the differentiated and undifferentiated cells according to Clontech protocols. These librarys were used to determine the relative abundance of Icrac-Mlsnl cDNA in the librarys.

Two nested primer pairs were designed to screen for Icrac-Mlsnl cDNA in the above librarys. The sequence of these primers is as follows:
Primer 1a (SEQ ID N°44) : TGAAGTAAAATCAATATCCAACCAGG
Primer 1b (SEQ ID N°45) : GCACTGCTCCTCGAACTCATGCAGCC
Primer 2a (SEQ ID N°46) : TCCAACCAGGTGTGGAAGTTCCAGCG
Primer 2b (SEQ ID N°47) : GGAAGTGCTCCTGCACGCACTGCTCC
Under the following PCR conditions:

All PCR reactions were carried out using Advantage cDNA PCR Kit (#K1905-1) in the suppliers buffer conditions. PCR primer concentration was 20 fmol/primer/100µl reaction. cDNA template concentration was 0.2µg. PCR reactions were carried out as two step cyles (denaturation 95°C for 30 secs, for annealing/elongation 68°C for 6min). PCR product was visualised by electrophoresis in an agarose gel and staining with ethidium bromide (0.5µg/ml).

Nested PCR using primer pair SEQ ID N°44 and 47 and nested pair SEQ ID N°45 and 46 indicated Mlsnl expression in U937 cells. Similar experiments indicated the expression of Mlsnl in Jurkat and KU-812 cells.

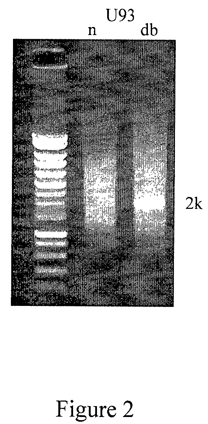

The presence of the expression of Icrac-Mlsnl in these librarys was further confirmed using nested PCR primers, SEQ ID NO: 44 and 46 together with CDS III/3 primer of SMART cDNA librarys (c.f. Advantage cDNA PCR Kit (#K1905-1)). A 2kb PCR product was obtained (figure 2). This corresponded to the predicted size of an Mlsnl PCR product and indicated that the gene encoding Mlsnl was being expressed in differentiated and undifferentiated U937 cell lines. However, the intensity of the band corresponding to the Mlsnl PCR product was greater in the differentiated cell library than in the undifferentiated cell library. These results indicate that Mlsnl cDNA was present in U937 cell lines and that this Mlsnl expression was more pronounced in U937 cell lines differentiated to a macrophage phenotype.

### 2. MLSNl(TrpC8) expression is differentially increased in dbcAMP treated (macrophage-like) U937 cells

The relative abundance of nucleic acid encoding Icrac-Mlsnl in undifferentiated and dbcAMP differentiated U937 cell lines was determined. Briefly, undifferentiated and dbcAMP differentiated U937 cell lines were cultured and harvested as described above, and subtracted cDNA libraries prepared using Clontech PCR Select kit (K1804-1) and the protocols therein. This technology allows for selective enrichment of differentially expressed genes between the differentiated and undifferentiated cell lines.

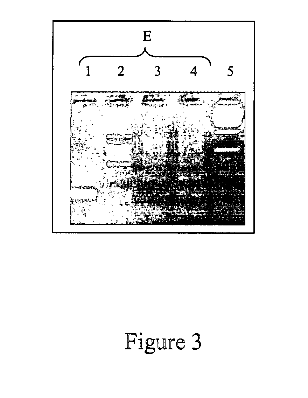

Following PCR substraction, Mlsnl specific primer pair (SEQ ID N°44 and 47) were used to amplify cDNA-fragments from Mlsnl from subtracted pools and controles using Advantage cDNA PCR, under suppliers conditions as two step cycles (95°C for 30 secs, 68°C for 1min annealing/elongation) (figure 3).

Forward subtraction of differentiated cells nucleic acid with non differentiated cell nucleic acid resulted in a strong Mlsnl specific PCR product. Back subtraction of non differentiated cells with differentiated cells resulted in undetectable amounts PCR product. This result indicates that there was greater Mlsnl gene expression in the differentiated U937 cells than in the undifferentiated U937 cells. This results correlates with the observation that the Icrac phenotype increases during dbcAMP treatment of U937 cell line to a more macrophage like phenotype. The inventors conclude that the emergence of an Icrac phenotype in the dbcAMP differentiated U937 cells corresponds with an increase in expression of Mlsnl.

### 3. Down regulation of calcium entry in dbcAMP treated (macrophage-like) U937 cells using Mlsnl anti-sense

U937 cells were cultured at 37°C in 5 % CO₂ in RPMI 1640, 10% heat inactivated FBS. Cells were incubated in absence or presence of 0, 1 or 10 µM Mlsnl anti-sense nucleic acid (SEQ ID NO:26), 4 hours prior to and 48h during differentiation treatment by dbcAMP. All oligonucleotides used in this experiment contained phosphorothiorate modifications at their first two and last wo linkages (terminal 5' and 3' linkage). A scrambled oligonucleotide (^{5'}CTGGTGGAAGAAGAGGACGTCCAT^{3'} ,SEQ ID N°48) was used at 10 µM in the same conditions as negative control. Another oligonucleotide against FcγRII (CD 32) (^{5'}TCTGGGACATACATTCTGAGACAT^{3'}, SEQ ID N° 49) was used at 10 µM in the same conditions as positive control for anti-sense uptake. DbcAMP induced differentiation was controlled by immunofluorescence using FACS quantitation of CD 32 and CD 64, as well as Ca²⁺ signaling after Thapsigargin stimulation, before and after dbcAMP treatment using fura 2 and fluorimetry. Intra-cellular Ca²⁺ after stimulation by ionomycin was measured during FACS analysis. Cells were loaded with 2 µg/ml indo-1 during 30 min at 37°C, 5 % CO₂ in RPMI, 2 mg/ml BSA. Cells were then activated by 2 µg/ml ionomycin. Ionomycin induced variations of intra-cellular calcium concentration were measured by flow cytometry (figure 4). CD 32 expression was quantitated by immunofluoresence with an FITC labeled anti CD32 antibody using flow cytometry.

DbcAMP treatment was shown to increase intra-cellular Ca²⁺ after stimulation according to Floto et al.. The results demonstrate that anti-sense oligonucleotide treated cultures have their calcium signal decreased by about 50%. This antisense experiment demonstrates that the down-regulation of Mlsnl expression anti-sense oligonucleotides results in a diminished Icrac phenotype in U937 cell lines. This result also demonstrates that anti-sense nucleic acid specific to Mlsnl is capable of down-regulating the Icrac phenotype.

### Reference List

1. Lewis, R.S. 1999. Store-operated calcium channels. *Adv Second Messenger Phosphoprotein Res 33*:279.
2. Berridge, M.J. 1993. Inositol trisphosphate and calcium signalling. *Nature 361*:315.
3. Streb, H., R.F. Irvine, M.J. Berridge, and I. Schulz. 1983. Release of Ca2+ from a nonmitochondrial intra-cellular store in pancreatic acinar cells by inositol-1,4,5-trisphosphate. *Nature 306*:67.
4. Putney, J.W.J. 1990. Capacitative calcium entry revisited. *Cell Calcium 11*:611.
5. Penner, R., G. Matthews, and E. Neher. 1988. Regulation of calcium influx by second messengers in rat mast cells. *Nature 334*:499.
6. Lewis, R.S. and M.D. Cahalan. 1989. Mitogen-induced oscillations of cytosolic Ca2+ and transmembrane Ca2+ current in human leukemic T-cells. *Cell Regul 1:*99.
7. Thastrup, O., P.J. Cullen, B.K. Drobak, M.R. Hanley, and A.P. Dawson. 1990. Thapsigargin, a tumor promoter, discharges intra-cellular Ca2+ stores by specific inhibition of the endoplasmic reticulum Ca²⁺-ATPase. *Proc Natl Acad Sci U S A 87*:2466.
8. Gouy, H., D. Cefai, S.B. Christensen, P. Debre, and G. Bismuth. 1990. Ca2+ influx in human T lymphocytes is induced independently of inositol phosphate production by mobilization of intra-cellular Ca²⁺ stores. A study with the Ca²⁺ endoplasmic reticulum-ATPase inhibitor thapsigargin. *Eur J Immunol 20*:2269.
9. Mason, M.J., M.P. Mahaut-Smith, and S. Grinstein. 1991. The role of intra-cellular Ca2+ in the regulation of the plasma membrane Ca²⁺ permeability of unstimulated rat lymphocytes. *J Biol Chem 266*:10872.
10. Hoth, M. and R. Penner. 1992. Depletion of intra-cellular calcium stores activates a calcium current in mast [see comments]. *Nature 355*:353.
11. Hoth, M. and R. Penner. 1993. Calcium release-activated calcium current in rat mastcells. *J Physiol (Lond) 465:*359.
12. Zweifach, A. and R.S. Lewis. 1993. Mitogen-regulated Ca2+ current of T lymphocytes is activated by depletion of intra-cellular Ca²⁺stores. *Proc Natl Acad Sci U S A 90:*6295.
13. Parekh, A.B. and R. Penner. 1997. Store depletion and calcium influx. *Physiol Rev 77*:901.
14. Clapham, D.E. 1995. Calcium signaling. *Cell 80:*259.
15. Dolmetsch, R.E., K. Xu, and R.S. Lewis. 1998. Calcium oscillations increase the efficiency and specificity of gene expression [see comments]. *Nature 392*:933.
16. Li, W., J. Llopis, M. Whitney, G. Zlokarnik, and R.Y. Tsien. 1998. Cell-permeant caged InsP3 ester shows that Ca2+ spike frequency can optimize gene expression [see comments]. *Nature 392*:936.
17. Partiseti, M., F. Le Deist, C. Hivroz, A. Fischer, H. Korn, and D. Choquet. 1994. The calcium current activated by T-cell receptor and store depletion in human lymphocytes is absent in a primary immunodeficiency. *J Biol Chem 269*:32327.
18. Floto RA, Mahaut-Smith MP, Allen JM, Somasundaram B.1996. Differentiation of the human monocytic cell line U937 results in an upregulation of the calcium release-activated current, ICRAC. *J*. *Physiol*. *(Lond)*. *495 : 331.*
19. Melendez A, Floto RA, Cameron AJ, Gillooly DJ, Harnett MM, Allen JM. 1998. A molecular switch changes the signalling pathway used by the Fc gamma RI antibody receptor to mobilise calcium. *Curr Biol*. *4 : 210.*

## Claims

1. The use of any purified or isolated nucleic acid encoding a human Icrac-Mlsnl or a sequence complementary thereto for the screening of Icrac-Mlsnl modulators, wherein the encoding nucleic acid encodes a polypeptide having the following characteristics:
(1) the N-terminal region is encoded by a consecutive sequence of at least 75 nucleotides, located 5' with respect to nucleotides encoding the transmembrane and C- terminal regions of Icrac-Mlsnl protein,
(2) a transmembrane region encoding six membrane spanning domains, encoded by a consecutive sequence of at least 150 nucleotides, located 3' with respect to the nucleotides encoding the N-terminal region and 5' with respect to nucleotides encoding the C-terminal region, and
(3) a C-terminal region encoded by a consecutive sequence of at least 75 nucleotides, located 3' with respect to nucleotides encoding the N-terminal region and transmembrane region.

2. The use of any purified or isolated nucleic acid encoding human Icrac-Mlsnl according to claim 1, wherein said nucleic acid has at least 80% nucleotide identity with the nucleotide sequence of SEQ ID N°23 or a sequence complementary thereto.

3. The use of any purified or isolated human Icrac-Mlsnl polypeptide or a fragment thereof, for the screening of Icrac-Mlsnl modulators, wherein the polypeptide has the following characteristics:
(1) the N-terminal region comprises a consecutive sequence of at least 25 amino acids, located N-terminal with respect to the transmembrane and C- terminal regions of Icrac-Mlsnl protein,
(2) a transmembrane region forming six membrane spanning domains, comprising a consecutive sequence of at least 50 amino acids, located C-terminal with respect to the N-terminal region and N-terminal with respect to the C-terminal region, and
(3) a C-terminal region comprising a consecutive sequence of at least 25 amino acids, located C-terminal with respect to the N-terminal and transmembrane regions.

4. The use of any purified or isolated polypeptide encoding human Icrac-Mlsnl according to claim 3, wherein said polypeptide has at least 90% amino identity with the sequence of SEQ ID N° 1 or a fragment thereof.

5. The use of any purified or isolated nucleic acid encoding a human Icrac-Mlsnl or a sequence complementary thereto for the diagnosis of disorders associated with aberrant Icrac function in immune cells, wherein the encoding nucleic acid encodes a polypeptide having the following characteristics:
(1) the N-terminal region is encoded by a consecutive sequence of at least 75 nucleotides, located 5' with respect to nucleotides encoding the transmembrane and C- terminal regions of Icrac-Mlsnl protein,
(2) a transmembrane region encoding six membrane spanning domains, encoded by a consecutive sequence of at least 150 nucleotides, located 3' with respect to the nucleotides encoding the N-terminal region and 5' with respect to nucleotides encoding the C-terminal region, and
(3) a C-terminal region encoded by a consecutive sequence of at least 75 nucleotides, located 3' with respect to nucleotides encoding the N-terminal region and transmembrane region.

6. The use of any purified or isolated nucleic acid encoding human Icrac-Mlsnl according to claim 5, wherein said nucleic acid has at least 80% nucleotide identity with the nucleotide sequence of SEQ ID N°23 or a sequence complementary thereto.

7. The use of any purified or isolated polypeptide encoding a human Icrac-Mlsnl or fragment thereof for the diagnosis of disorders associated with aberrant Icrac function in immune cells, wherein the polypeptide has the following characteristics:
(1) the N-terminal region comprises a consecutive sequence of at least 25 amino acids, located N-terminal with respect to the transmembrane and C- terminal regions of Icrac-Mlsnl protein,
(2) a transmembrane region forming six membrane spanning domains, comprising a consecutive sequence of at least 50 amino acids, located C-terminal with respect to the N-terminal region and N-terminal with respect to the C-terminal region, and
(3) a C-terminal region comprising a consecutive sequence of at least 25 amino acids, located C-terminal with respect to the N-terminal and transmembrane regions.

8. The use of any purified or isolated polypeptide encoding human Icrac-Mlsnl according to claim 7, wherein said polypeptide has at least 90% amino identity with the sequence of SEQ ID N° 1 or a fragment thereof.

9. A purified or isolated human Icrac-Mlsnl N-terminal polypeptide region, wherein the N-terminal region comprises a consecutive sequence of at least 25 amino acids, located N-terminal of the first transmembrane region of the Icrac-Mlsnl protein.

10. A purified or isolated human Icrac-Mlsnl N-terminal polypeptide region according to claim 9, wherein said N-terminal polypeptide has at least 90% amino acid identity with any of the amino acid sequences SEQ ID N°2 or 3, or fragments thereof.

11. A purified or isolated human Icrac-Mlsnl C-terminal polypeptide region, wherein the C-terminal region comprises a consecutive sequence of at least 25 amino acids, located C-terminal of the sixth transmembrane region of the Icrac-Mlsnl protein.

12. A purified or isolated human Icrac-Mlsnl C-terminal region according to claim 11, wherein said C-terminal polypeptide has at least 90% amino acid identity with any of the polypeptides of amino acid sequences SEQ ID N°4 or 5, or fragments thereof.

13. A purified or isolated human Icrac-Mlsnl Intra-cellular loop-1 polypeptide region, wherein the intra-cellular loop-1 region comprises a consecutive sequence of at least 7 amino acids, located C-terminal of the second transmembrane region and N-terminal to the third transmembrane region of the Icrac-Mlsnl protein.

14. A purified or isolated human Icrac-Mlsnl intracellular loop-1 region according to claim 13, wherein said intra-cellular loop-1 polypeptide has at least 90% amino acid identity with any of the polypeptides of amino acid sequences SEQ ID N°6 or 7, or fragments thereof.

15. A purified or isolated human Icrac-Mlsnl intra-cellular loop-2 polypeptide region, wherein the intra-cellular loop-2 polypeptide region comprises a consecutive sequence of at least 7 amino acids, located C-terminal of the fourth transmembrane region and N-terminal of the fifth transmembrane region of the Icrac-Mlsnl protein.

16. A purified or isolated human Icrac-Mlsnl intra-cellular loop-2 region according to claim 15, wherein said intra-cellular loop-2 region polypeptide has at least 90% amino acid identity with any of the polypeptides of amino acid sequences SEQ ID N°8 or 9, or fragments thereof.

17. A purified or isolated human Icrac-Mlsnl extra-cellular loop-1 polypeptide region, wherein the extra-cellular loop-1 region comprises a consecutive sequence of at least 5 amino acids, located C-terminal of the first transmembrane region and N-terminal of the second transmembrane region of the Icrac-Mlsnl protein.

18. A purified or isolated human Icrac-Mlsnl extra-cellular loop-1 region according to claim 17, wherein said extra-cellular loop-1 region polypeptide has at least 90% amino acid identity with any of the polypeptides of amino acid sequences SEQ ID N°10 or 11, or fragments thereof.

19. A purified or isolated human Icrac-Mlsnl extra-cellular loop-2 polypeptide region, wherein the extra-cellular loop-2 region comprises a consecutive sequence of at least 5 amino acids, located C-terminal of the third transmembrane region and N-terminal of the fourth transmembrane region of the Icrac-Mlsnl protein.

20. A purified or isolated human Icrac-Mlsnl extra-cellular loop-2 region according to claim 19, wherein said extra-cellular loop-2 region polypeptide has at least 90% amino acid identity with any of the polypeptides of amino acid sequences SEQ ID N°12 or 13, or fragments thereof.

21. A purified or isolated human Icrac-Mlsnl extra-cellular loop-3 polypeptide region, wherein the extra-cellular loop-3 region comprises a consecutive sequence of at least 7 amino acids C-terminal of the fifth transmembrane region and N-terminal of the sixth transmembrane region of the Icrac-Mlsnl protein.

22. A purified or isolated human Icrac-Mlsnl extra-cellular loop-3 region according to claim 21, wherein said extra-cellular loop-3 region polypeptide has at least 90% amino acid identity with any of the polypeptides of amino acid sequences SEQ ID N°14, 15, 16 or 17, or fragments thereof.

23. A purified or isolated human Icrac-Mlsnl polypeptide containing both intra- and extra-cellular loop regions, wherein said intra-and extra-cellular loop region comprises a consecutive sequence of at least 50 amino acids, located C-terminal to the N-terminal region and N-terminal to the C-terminal region of the Icrac-Mlsnl protein.

24. A purified or isolated human Icrac-Mlsnl peptide containing both intra-and extra-cellular loop regions according to claim 23, wherein said intra- and extra-cellular loop region polypeptide has at least 90% amino acid identity with any of the polypeptides of amino acid sequences SEQ ID N° 18 or 19, or fragments thereof.

25. A purified or isolated nucleic acid encoding the human Icrac-Mlsnl N-terminal region, wherein the N-terminal region is encoded by a consecutive sequence of at least 75 nucleotides, located 5' with respect to nucleotides encoding the first transmembrane region of the Icrac-Mlsnl protein.

26. A purified or isolated nucleic acid encoding the human Icrac-Mlsnl N-terminal region according to claim 25, wherein said nucleic acid has at least 80% nucleic acid identity with any of the nucleotide sequences SEQ ID N°24 or 25, or a complementary sequence thereto.

27. A purified or isolated nucleic acid encoding the human Icrac-Mlsnl C-terminal region, wherein the C-terminal region is encoded by a consecutive sequence of at least 75 nucleotides, located 5' with respect to nucleotides encoding the sixth transmembrane region of the Icrac-Mlsnl protein.

28. A purified or isolated nucleic acid encoding the human Icrac-Mlsnl C-terminal region according to claim 27, wherein said nucleic acid has at least 80% nucleic acid identity with any of the nucleotide sequences of SEQ ID N°26 or 27, or a complementary sequence thereto.

29. A purified or isolated nucleic acid encoding the human Icrac-Mlsnl intra-cellular loop-1 region, wherein the intra-cellular loop-1 region is encoded by a consecutive sequence of at least 21 nucleotides, located 3' with respect to nucleotides encoding the second transmembrane region and 5' with respect to nucleotides encoding the third transmembrane region of the Icrac-Mlsnl protein.

30. A purified or isolated nucleic acid encoding the human Icrac-Mlsnl intra-cellular loop-1 region according to claim 29, wherein said nucleic acid has at least 80% nucleic acid identity with any of the nucleotide sequences of SEQ ID N°28 or 29, or a complementary sequence thereto.

31. A purified or isolated nucleic acid encoding the human Icrac-Mlsnl intra-cellular loop-2 region, wherein the intra-cellular loops-2 region is encoded by a consecutive sequence of at least 21 nucleotides, located 3' with respect to nucleotides encoding the fourth transmembrane region and 5' with respect to nucleotides encoding the fifth transmembrane region of the Icrac-Mlsnl protein.

32. A purified or isolated nucleic acid encoding the human Icrac-Mlsnl intra-cellular loop-2 region according to claim 31, wherein said nucleic acid has at least 80% nucleic acid identity with any of the nucleotide sequences of SEQ ID N°30 or 31, or a complementary sequence thereto.

33. A purified or isolated nucleic acid encoding the human Icrac-Mlsnl extra-cellular loop-1 region, wherein the extra-cellular loop-1 region is encoded by a consecutive sequence of at least 15 nucleotides, located 3' with respect to nucleotides encoding the first transmembrane region and 5' with respect to nucleotides encoding the second transmembrane region of the Icrac-Mlsnl protein.

34. A purified or isolated nucleic acid encoding the human Icrac-Mlsnl extra-cellular loop-1 region according to claim 33, wherein said nucleic acid has at least 80% nucleic acid identity with any of the nucleotide sequences of SEQ ID N°32 or 33, or a complementary sequence thereto.

35. A purified or isolated nucleic acid encoding the human Icrac-Mlsnl extra-cellular loop-2 region, wherein the extra-cellular loop-2 region is encoded by a consecutive sequence of at least 15 nucleotides, located 3' with respect to nucleotides encoding the third transmembrane region and 5' with respect to nucleotides encoding the fourth transmembrane region of the Icrac-Mlsnl protein.

36. A purified or isolated nucleic acid encoding the human Icrac-Mlsnl extra-cellular loop-2 region according to claim 35, wherein said nucleic acid has at least 80% nucleic acid identity with any of the nucleotide sequences of SEQ ID N°34 or 35, or a complementary sequence thereto.

37. A purified or isolated nucleic acid encoding the human Icrac-Mlsnl extra-cellular loop-3 region, wherein the extra-cellular loop-3 region is encoded by a consecutive sequence of at least 21 nucleotides, located 3' with respect to nucleotides encoding the fifth transmembrane region and 5' with respect to nucleotides encoding the sixth transmembrane region of the Icrac-Mlsnl protein.

38. A purified or isolated nucleic acid encoding the human Icrac-Mlsnl extra-cellular loop-3 region according to claim 37, wherein said nucleic acid has at least 80% nucleic acid identity with any of the nucleotide sequences of SEQ ID N°36, 37 or 38, or a complementary sequence thereto.

39. A purified or isolated nucleic acid encoding a human Icrac-Mlsnl polypeptide containing both intra- and extra-cellular loop regions, wherein said intra- and extra-cellular loop region is encoded by a consecutive sequence of at least 150 nucleotides, located 3' with respect to nucleotides encoding the N-terminal region and 5' with respect to nucleotides encoding the C-terminal regions of the Icrac-Mlsnl protein.

40. A purified or isolated nucleic acid encoding the human Icrac-Mlsnl polypeptide containing both intra- and extra-cellular loop regions according to claim 39, wherein said nucleic acid has at least 80% nucleic acid identity with any of the nucleotide sequences of SEQ ID N°39 or 40, or a complementary sequence thereto.

41. A nucleic acid sequence encoding an Icrac-Mlsnl transcription promoter of SEQ ID N°41 or a complementary sequence thereto.

42. A nucleic acid sequence of claim 41, having at least 80% nucleotide identity with the nucleotide sequence of SEQ ID N°41 or a complementary sequence thereto.

43. A nucleic acid sequence encoding an Icrac-Mlsnl transcription promoter of SEQ ID N°42 or a complementary sequence thereto.

44. A nucleic acid sequence of claim 43, having at least 80% nucleotide identity with the nucleotide sequence of SEQ ID N°42 or a complementary sequence thereto.

45. A nucleic acid sequence encoding an Icrac-Mlsnl transcription promoter of SEQ ID N°43 or a complementary sequence thereto.

46. A nucleic acid sequence of claim 45, having at least 80% nucleotide identity with the nucleotide sequence of SEQ ID N°43 or a complementary sequence thereto.

47. A recombinant vector comprising a nucleic acid according to any one of the claims 25 to 46.

48. A recombinant vector comprising a nucleic acid encoding an Mlsnl polypeptide according to any one of the claims 9 to24

49. A recombinant host cell comprising a nucleic acid according to any one of the claims 25 to 46 or a vector of claim 47 or 48.

50. A recombinant host cell comprising a nucleic acid ncoding an Mlsnl polypeptide according to any one of the claims 9 to 24.

51. A method for producing a Mlsnl polypeptide, wherein the said method comprises the following steps of:
a) culturing, in an appropriate culture medium, a host cell previously transformed or transfected with a polynucleotide according to any one of claims 25 to 48,
b) harvesting the culture medium thus conditioned or lyse the host cell, for example by sonication or by osmotic shock ; and
c) separating or purifying, from said culture medium, or from the pellet of the resulting cell lysate, the produced Mlsnl polypeptide of interest.

52. A method for screening ligand substances or molecules that are able to bind to a Icrac-Mlsnl, or fragment thereof, or Mlsnl transcription promoter elements of any one of the claims 1 to 46, said method comprising ;
a) contacting a test compound with Icrac-Mlsnl gene, an Icrac-Mlsnl gene product or Icrac-Mlsnl transcription promoter element and,
b) measuring the ability of said test compound to interact with the Icrac-Mlsnl gene, Icrac-Mlsnl gene product or transcription promoter element (i.e., binding assay).

53. A method for screening ligand substances or molecules that are able to modulate the biological activity of Icrac-Mlsnl, or fragment thereof, of any one of the claims 1 to 40, said method comprising ;
a) contacting a test compound with an Icrac-Mlsnl expressing cell, and,
b) determining the ability of said test compound to affect Icrac-Mlsnl gene or an Icrac-Mlsnl gene product activity within the cells.

54. A method for screening ligand substances or molecules that are able to modulate the biological activity of Icrac-Mlsnl, or fragment thereof, of any one of the claims 1 to 40, said method comprising ;
a) obtaining a recombinant cell expressing Icrac-Mlsnl gene or gene product,
b) exposing said recombinant cell to a substance or molecule to be tested ; and
c) Measuring the change in calcium flux or activation potential within the exposed recombinant cell.

55. A method for screening ligand substances or molecules that are able to modulate the biological activity of Icrac-Mlsnl, or fragment thereof, of any one of the claims 41 to 46, said method comprising ;
a) obtaining a recombinant cell expressing a reporter molecule under the control of an Icrac-Mlsnl transcription promoterIcrac-Mlsnl gene,
b) Exposing said recombinant cell to a substance or molecule to be tested ; and
c) Measuring the change in reporter expression or activity in said recombinant cell.

56. A method for screening ligand substances or molecules that are able to bind to a Icrac-Mlsnl, or modulate the biological activity of Icrac-Mlsnl, according to any one of claims 51 to 55, wherein a competitor Mlsnl binding ligand, preferably selected from the class of pyrazol compounds such as SEW04225, KM02940, KM03000 or GK02421, is added and the binding of said competitor in the presence and/or absence of a test compound is determined.

57. A method for detecting any one of the nucleic acids of claims 6, 8, or 25 to 50, in a blood sample, said method comprising ;
a) obtaining a blood sample,
b) optionally separating immune cells from other blood components,
c) reverse transcribing cellular RNA to cDNA, and
d) amplifying Mlsnl nucleic acid obtained in step c) by PCR with a plurality of Mlsnl specific oligonucleotides capable of hybridizing, under stringent conditions to Mlsnl nucleic acids and characterize the PCR product.

58. The use of a Icrac-Mlsnl gene, gene product or transcriptional element, to screen compounds that modulate the activity of an antigen presenting cell, particularly of macrophages, in particular macrophage maturation.

59. The use of a compound that modulates the activity of Icrac-Mlsnl gene, gene product or transcriptional element, for the manufacture of a composition to regulate the activity of an antigen presenting cell, particularly of macrophages, in particular macrophage maturation.

60. An antisense nucleic acid molecule comprising a sequence region which is complementary to at least a region of a Icrac-Mlsnl nucleic acid of claims 25 to 40.


SEQUENCE LISTING

<110> Warner-Lambert

<120> IDENTIFICATION OF THE MAJOR CAPACITATIVE CALCIUM
      CHANNEL IN ANTIGEN PRESENTING CELLS AND USES THEREOF

<130> Icrac-Mlsn1(310800)

<140>
<141>

<160> 49

<170> PatentIn Ver. 2.1

<210> 1
<211> 1533
<212> PRT
<213> Homo sapiens

<400> 1


<210> 2
<211> 756
<212> PRT
<213> Homo sapiens

<400> 2
Met Tyr Ile Arg Val Ser Tyr Asp Thr Lys Pro Asp Ser Leu Leu His
  1               5                  10                  15

Leu Met Val Lys Asp Trp Gln Leu Glu Leu Pro Lys Leu Leu Ile Ser
             20                  25                  30

Val His Gly Gly Leu Gln Asn Phe Glu Met Gln Pro Lys Leu Lys Gln
         35                  40                  45

Val Phe Gly Lys Gly Leu Ile Lys Ala Ala Met Thr Thr Gly Ala Trp
     50                  55                  60

Ile Phe Thr Gly Gly Val Ser Thr Gly Val Ile Ser His Val Gly Asp
 65                  70                  75                  80

Ala Leu Lys Asp His Ser Ser Lys Ser Arg Gly Arg Val Cys Ala Ile
                 85                  90                  95

Gly Ile Ala Pro Trp Gly Ile Val Glu Asn Lys Glu Asp Leu Val Gly
            100                 105                 110

Lys Asp Val Thr Arg Val Tyr Gln Thr Met Ser Asn Pro Leu Ser Lys
        115                 120                 125

Leu Ser Val Leu Asn Asn Ser His Thr His Phe Ile Leu Ala Asp Asn
    130                 135                 140

Gly Thr Leu Gly Lys Tyr Gly Ala Glu Val Lys Leu Arg Arg Leu Leu
145                 150                 155                 160

Glu Lys His Ile Ser Leu Gln Lys Ile Asn Thr Arg Leu Gly Gln Gly
                165                 170                 175

Val Pro Leu Val Gly Leu Val Val Glu Gly Gly Pro Asn Val Val Ser
            180                 185                 190

Ile Val Leu Glu Tyr Leu Gln Glu Glu Pro Pro Ile Pro Val Val Ile
        195                 200                 205

Cys Asp Gly Ser Gly Arg Ala Ser Asp Ile Leu Ser Phe Ala His Lys
    210                 215                 220

Tyr Cys Glu Glu Gly Gly Ile Ile Asn Glu Ser Leu Arg Glu Gln Leu

225                 230                 235                 240

Leu Val Thr Ile Gln Lys Thr Phe Asn Tyr Asn Lys Ala Gln Ser His
                245                 250                 255

Gln Leu Phe Ala Ile Ile Met Glu Cys Met Lys Lys Lys Glu Leu Val
            260                 265                 270

Thr Val Phe Arg Met Gly Ser Glu Gly Gln Gln Asp Ile Glu Met Ala
        275                 280                 285

Ile Leu Thr Ala Leu Leu Lys Gly Thr Asn Val Ser Ala Pro Asp Gln
    290                 295                 300

Leu Ser Leu Ala Leu Ala Trp Asn Arg Val Asp Ile Ala Arg Ser Gln
305                 310                 315                 320

Ile Phe Val Phe Gly Pro His Trp Thr Pro Leu Gly Ser Leu Ala Pro
                325                 330                 335

Pro Thr Asp Ser Lys Ala Thr Glu Lys Glu Lys Lys Pro Pro Met Ala
            340                 345                 350

Thr Thr Lys Gly Gly Arg Gly Lys Gly Lys Gly Lys Lys Lys Gly Lys
        355                 360                 365

Val Lys Glu Glu Val Glu Glu Glu Thr Asp Pro Arg Lys Ile Glu Leu
    370                 375                 380

Leu Asn Trp Val Asn Ala Leu Glu Gln Ala Met Leu Asp Ala Leu Val
385                 390                 395                 400

Leu Asp Arg Val Asp Phe Val Lys Leu Leu Ile Glu Asn Gly Val Asn
                405                 410                 415

Met Gln His Phe Leu Thr Ile Pro Arg Leu Glu Glu Leu Tyr Asn Thr
            420                 425                 430

Arg Leu Gly Pro Pro Asn Thr Leu His Leu Leu Val Arg Asp Val Lys
        435                 440                 445

Lys Ser Asn Leu Pro Pro Asp Tyr His Ile Ser Leu Ile Asp Ile Gly
    450                 455                 460

Leu Val Leu Glu Tyr Leu Met Gly Gly Ala Tyr Arg Cys Asn Tyr Thr
465                 470                 475                 480

Arg Lys Asn Phe Arg Thr Leu Tyr Asn Asn Leu Phe Gly Pro Lys Arg
                485                 490                 495

Pro Lys Ala Leu Lys Leu Leu Gly Met Glu Asp Asp Glu Pro Pro Ala
            500                 505                 510

Lys Gly Lys Lys Lys Lys Lys Lys Lys Lys Glu Glu Glu Ile Asp Ile
        515                 520                 525

Asp Val Asp Asp Pro Ala Val Ser Arg Phe Gln Tyr Pro Phe His Glu
    530                 535                 540

Leu Met Val Trp Ala Val Leu Met Lys Arg Gln Lys Met Ala Val Phe

545                 550                 555                 560

Leu Trp Gln Arg Gly Glu Glu Ser Met Ala Lys Ala Leu Val Ala Cys
                565                 570                 575

Lys Leu Tyr Lys Ala Met Ala His Glu Ser Ser Glu Ser Asp Leu Val
            580                 585                 590

Asp Asp Ile Ser Gln Asp Leu Asp Asn Asn Ser Lys Asp Phe Gly Gln
        595                 600                 605

Leu Ala Leu Glu Leu Leu Asp Gln Ser Tyr Lys His Asp Glu Gln Ile
    610                 615                 620

Ala Met Lys Leu Leu Thr Tyr Glu Leu Lys Asn Trp Ser Asn Ser Thr
625                 630                 635                 640

Cys Leu Lys Leu Ala Val Ala Ala Lys His Arg Asp Phe Ile Ala His
                645                 650                 655

Thr Cys Ser Gln Met Leu Leu Thr Asp Met Trp Met Gly Arg Leu Arg
            660                 665                 670

Met Arg Lys Asn Pro Gly Leu Lys Val Ile Met Gly Ile Leu Leu Pro
        675                 680                 685

Pro Thr Ile Leu Phe Leu Glu Phe Arg Thr Tyr Asp Asp Phe Ser Tyr
    690                 695                 700

Gln Thr Ser Lys Glu Asn Glu Asp Gly Lys Glu Lys Glu Glu Glu Asn
705                 710                 715                 720

Thr Asp Ala Asn Ala Asp Ala Gly Ser Arg Lys Gly Asp Glu Glu Asn
                725                 730                 735

Glu His Lys Lys Gln Arg Ser Ile Pro Ile Gly Thr Lys Ile Cys Glu
            740                 745                 750

Phe Tyr Asn Ala
        755


<210> 3
<211> 761
<212> PRT
<213> Homo sapiens

<400> 3
Met Tyr Ile Arg Val Ser Tyr Asp Thr Lys Pro Asp Ser Leu Leu His
1               5                   10                  15

Leu Met Val Lys Asp Trp Gln Leu Glu Leu Pro Lys Leu Leu Ile Ser
            20                  25                  30

Val His Gly Gly Leu Gln Asn Phe Glu Met Gln Pro Lys Leu Lys Gln
        35                  40                  45

Val Phe Gly Lys Gly Leu Ile Lys Ala Ala Met Thr Thr Gly Ala Trp
    50                  55                  60

Ile Phe Thr Gly Gly Val Ser Thr Gly Val Ile Ser His Val Gly Asp
65                  70                  75                  80

Ala Leu Lys Asp His Ser Ser Lys Ser Arg Gly Arg Val Cys Ala Ile
                85                  90                  95

Gly Ile Ala Pro Trp Gly Ile Val Glu Asn Lys Glu Asp Leu Val Gly
            100                 105                 110

Lys Asp Val Thr Arg Val Tyr Gln Thr Met Ser Asn Pro Leu Ser Lys
        115                 120                 125

Leu Ser Val Leu Asn Asn Ser His Thr His Phe Ile Leu Ala Asp Asn
    130                 135                 140

Gly Thr Leu Gly Lys Tyr Gly Ala Glu Val Lys Leu Arg Arg Leu Leu
145                 150                 155                 160

Glu Lys His Ile Ser Leu Gln Lys Ile Asn Thr Arg Leu Gly Gln Gly
                165                 170                 175

Val Pro Leu Val Gly Leu Val Val Glu Gly Gly Pro Asn Val Val Ser
            180                 185                 190

Ile Val Leu Glu Tyr Leu Gln Glu Glu Pro Pro Ile Pro Val Val Ile
        195                 200                 205

Cys Asp Gly Ser Gly Arg Ala Ser Asp Ile Leu Ser Phe Ala His Lys
    210                 215                 220

Tyr Cys Glu Glu Gly Gly Ile Ile Asn Glu Ser Leu Arg Glu Gln Leu
225                 230                 235                 240

Leu Val Thr Ile Gln Lys Thr Phe Asn Tyr Asn Lys Ala Gln Ser His
                245                 250                 255

Gln Leu Phe Ala Ile Ile Met Glu Cys Met Lys Lys Lys Glu Leu Val
            260                 265                 270

Thr Val Phe Arg Met Gly Ser Glu Gly Gln Gln Asp Ile Glu Met Ala
        275                 280                 285

Ile Leu Thr Ala Leu Leu Lys Gly Thr Asn Val Ser Ala Pro Asp Gln
    290                 295                 300

Leu Ser Leu Ala Leu Ala Trp Asn Arg Val Asp Ile Ala Arg Ser Gln
305                 310                 315                 320

Ile Phe Val Phe Gly Pro His Trp Thr Pro Leu Gly Ser Leu Ala Pro
                325                 330                 335

Pro Thr Asp Ser Lys Ala Thr Glu Lys Glu Lys Lys Pro Pro Met Ala
            340                 345                 350

Thr Thr Lys Gly Gly Arg Gly Lys Gly Lys Gly Lys Lys Lys Gly Lys
        355                 360                 365

Val Lys Glu Glu Val Glu Glu Glu Thr Asp Pro Arg Lys Ile Glu Leu
    370                 375                 380

Leu Asn Trp Val Asn Ala Leu Glu Gln Ala Met Leu Asp Ala Leu Val
385                 390                 395                 400

Leu Asp Arg Val Asp Phe Val Lys Leu Leu Ile Glu Asn Gly Val Asn
                405                 410                 415

Met Gln His Phe Leu Thr Ile Pro Arg Leu Glu Glu Leu Tyr Asn Thr
            420                 425                 430

Arg Leu Gly Pro Pro Asn Thr Leu His Leu Leu Val Arg Asp Val Lys
        435                 440                 445

Lys Ser Asn Leu Pro Pro Asp Tyr His Ile Ser Leu Ile Asp Ile Gly
    450                 455                 460

Leu Val Leu Glu Tyr Leu Met Gly Gly Ala Tyr Arg Cys Asn Tyr Thr
465                 470                 475                 480

Arg Lys Asn Phe Arg Thr Leu Tyr Asn Asn Leu Phe Gly Pro Lys Arg
                485                 490                 495

Pro Lys Ala Leu Lys Leu Leu Gly Met Glu Asp Asp Glu Pro Pro Ala
            500                 505                 510

Lys Gly Lys Lys Lys Lys Lys Lys Lys Lys Glu Glu Glu Ile Asp Ile
        515                 520                 525

Asp Val Asp Asp Pro Ala Val Ser Arg Phe Gln Tyr Pro Phe His Glu
    530                 535                 540

Leu Met Val Trp Ala Val Leu Met Lys Arg Gln Lys Met Ala Val Phe
545                 550                 555                 560

Leu Trp Gln Arg Gly Glu Glu Ser Met Ala Lys Ala Leu Val Ala Cys
                565                 570                 575

Lys Leu Tyr Lys Ala Met Ala His Glu Ser Ser Glu Ser Asp Leu Val
            580                 585                 590

Asp Asp Ile Ser Gln Asp Leu Asp Asn Asn Ser Lys Asp Phe Gly Gln
        595                 600                 605

Leu Ala Leu Glu Leu Leu Asp Gln Ser Tyr Lys His Asp Glu Gln Ile
    610                 615                 620

Ala Met Lys Leu Leu Thr Tyr Glu Leu Lys Asn Trp Ser Asn Ser Thr
625                 630                 635                 640

Cys Leu Lys Leu Ala Val Ala Ala Lys His Arg Asp Phe Ile Ala His
                645                 650                 655

Thr Cys Ser Gln Met Leu Leu Thr Asp Met Trp Met Gly Arg Leu Arg
            660                 665                 670

Met Arg Lys Asn Pro Gly Leu Lys Val Ile Met Gly Ile Leu Leu Pro
        675                 680                 685

Pro Thr Ile Leu Phe Leu Glu Phe Arg Thr Tyr Asp Asp Phe Ser Tyr
    690                 695                 700

Gln Thr Ser Lys Glu Asn Glu Asp Gly Lys Glu Lys Glu Glu Glu Asn
705                 710                 715                 720

Thr Asp Ala Asn Ala Asp Ala Gly Ser Arg Lys Gly Asp Glu Glu Asn
                725                 730                 735

Glu His Lys Lys Gln Arg Ser Ile Pro Ile Gly Thr Lys Ile Cys Glu
            740                 745                 750

Phe Tyr Asn Ala Pro Ile Val Lys Phe
        755                 760


<210> 4
<211> 518
<212> PRT
<213> Homo sapiens

<400> 4
Ser Asn Gln Val Trp Lys Phe Gln Arg Tyr Gln Leu Ile Met Thr Phe
  1               5                  10                  15

His Asp Arg Pro Val Leu Pro Pro Pro Met Ile Ile Leu Ser His Ile
             20                  25                  30

Tyr Ile Ile Ile Met Arg Leu Ser Gly Arg Cys Arg Lys Lys Arg Glu
         35                  40                  45

Gly Asp Gln Glu Glu Arg Asp Arg Gly Leu Lys Leu Phe Leu Ser Asp
     50                  55                  60

Glu Glu Leu Lys Arg Leu His Glu Phe Glu Glu Gln Cys Val Gln Glu
 65                  70                  75                  80

His Phe Arg Glu Lys Glu Asp Glu Gln Gln Ser Ser Ser Asp Glu Arg
                 85                  90                  95

Ile Arg Val Thr Ser Glu Arg Val Glu Asn Met Ser Met Arg Leu Glu
            100                 105                 110

Glu Ile Asn Glu Arg Glu Thr Phe Met Lys Thr Ser Leu Gln Thr Val
        115                 120                 125

Asp Leu Arg Leu Ala Gln Leu Glu Glu Leu Ser Asn Arg Met Val Asn
    130                 135                 140

Ala Leu Glu Asn Leu Ala Gly Ile Asp Arg Ser Asp Leu Ile Gln Ala
145                 150                 155                 160

Arg Ser Arg Ala Ser Ser Glu Cys Glu Ala Thr Tyr Leu Leu Arg Gln
                165                 170                 175

Ser Ser Ile Asn Ser Ala Asp Gly Tyr Ser Leu Tyr Arg Tyr His Phe
            180                 185                 190

Asn Gly Glu Glu Leu Leu Phe Glu Asp Thr Ser Leu Ser Thr Ser Pro
        195                 200                 205

Gly Thr Gly Val Arg Lys Lys Thr Cys Ser Phe Arg Ile Lys Glu Glu
    210                 215                 220

Lys Asp Val Lys Thr His Leu Val Pro Glu Cys Gln Asn Ser Leu His
225                 230                 235                 240

Leu Ser Leu Gly Thr Ser Thr Ser Ala Thr Pro Asp Gly Ser His Leu
                245                 250                 255

Ala Val Asp Asp Leu Lys Asn Ala Glu Glu Ser Lys Leu Gly Pro Asp
            260                 265                 270

Ile Gly Ile Ser Lys Glu Asp Asp Glu Arg Gln Thr Asp Ser Lys Lys
        275                 280                 285

Glu Glu Thr Ile Ser Pro Ser Leu Asn Lys Thr Asp Val Ile His Gly
    290                 295                 300

Gln Asp Lys Ser Asp Val Gln Asn Thr Gln Leu Thr Val Glu Thr Thr
305                 310                 315                 320

Asn Ile Glu Gly Thr Ile Ser Tyr Pro Leu Glu Glu Thr Lys Ile Thr
                325                 330                 335

Arg Tyr Phe Pro Asp Glu Thr Ile Asn Ala Cys Lys Thr Met Lys Ser
            340                 345                 350

Arg Ser Phe Val Tyr Ser Arg Gly Arg Lys Leu Val Gly Gly Val Asn
        355                 360                 365

Gln Asp Val Glu Tyr Ser Ser Ile Thr Asp Gln Gln Leu Thr Thr Glu
    370                 375                 380

Trp Gln Cys Gln Val Gln Lys Ile Thr Arg Ser His Ser Thr Asp Ile
385                 390                 395                 400

Pro Tyr Ile Val Ser Glu Ala Ala Val Gln Ala Glu Gln Lys Glu Gln
                405                 410                 415

Phe Ala Asp Met Gln Asp Glu His His Val Ala Glu Ala Ile Pro Arg
            420                 425                 430

Ile Pro Arg Leu Ser Leu Thr Ile Thr Asp Arg Asn Gly Met Glu Asn
        435                 440                 445

Leu Leu Ser Val Lys Pro Asp Gln Thr Leu Gly Phe Pro Ser Leu Arg
    450                 455                 460

Ser Lys Ser Leu His Gly His Pro Arg Asn Val Lys Ser Ile Gln Gly
465                 470                 475                 480

Lys Leu Asp Arg Ser Gly His Ala Ser Ser Val Ser Ser Leu Val Ile
                485                 490                 495

Val Ser Gly Met Thr Ala Glu Glu Lys Lys Val Lys Lys Glu Lys Ala
            500                 505                 510

Ser Thr Glu Thr Glu Cys
        515

<210> 5

<211> 524
<212> PRT
<213> Homo sapiens

<400> 5
Phe Glu Val Lys Ser Ile Ser Asn Gln Val Trp Lys Phe Gln Arg Tyr
1               5                   10                  15

Gln Leu Ile Met Thr Phe His Asp Arg Pro Val Leu Pro Pro Pro Met
            20                  25                  30

Ile Ile Leu Ser His Ile Tyr Ile Ile Ile Met Arg Leu Ser Gly Arg
        35                  40                  45

Cys Arg Lys Lys Arg Glu Gly Asp Gln Glu Glu Arg Asp Arg Gly Leu
    50                  55                  60

Lys Leu Phe Leu Ser Asp Glu Glu Leu Lys Arg Leu His Glu Phe Glu
65                  70                  75                  80

Glu Gln Cys Val Gln Glu His Phe Arg Glu Lys Glu Asp Glu Gln Gln
                85                  90                  95

Ser Ser Ser Asp Glu Arg Ile Arg Val Thr Ser Glu Arg Val Glu Asn
            100                 105                 110

Met Ser Met Arg Leu Glu Glu Ile Asn Glu Arg Glu Thr Phe Met Lys
        115                 120                 125

Thr Ser Leu Gln Thr Val Asp Leu Arg Leu Ala Gln Leu Glu Glu Leu
    130                 135                 140

Ser Asn Arg Met Val Asn Ala Leu Glu Asn Leu Ala Gly Ile Asp Arg
145                 150                 155                 160

Ser Asp Leu Ile Gln Ala Arg Ser Arg Ala Ser Ser Glu Cys Glu Ala
                165                 170                 175

Thr Tyr Leu Leu Arg Gln Ser Ser Ile Asn Ser Ala Asp Gly Tyr Ser
            180                 185                 190

Leu Tyr Arg Tyr His Phe Asn Gly Glu Glu Leu Leu Phe Glu Asp Thr
        195                 200                 205

Ser Leu Ser Thr Ser Pro Gly Thr Gly Val Arg Lys Lys Thr Cys Ser
    210                 215                 220

Phe Arg Ile Lys Glu Glu Lys Asp Val Lys Thr His Leu Val Pro Glu
225                 230                 235                 240

Cys Gln Asn Ser Leu His Leu Ser Leu Gly Thr Ser Thr Ser Ala Thr
                245                 250                 255

Pro Asp Gly Ser His Leu Ala Val Asp Asp Leu Lys Asn Ala Glu Glu
            260                 265                 270

Ser Lys Leu Gly Pro Asp Ile Gly Ile Ser Lys Glu Asp Asp Glu Arg
        275                 280                 285

Gln Thr Asp Ser Lys Lys Glu Glu Thr Ile Ser Pro Ser Leu Asn Lys

290                 295                 300

Thr Asp Val Ile His Gly Gln Asp Lys Ser Asp Val Gln Asn Thr Gln
305                 310                 315                 320

Leu Thr Val Glu Thr Thr Asn Ile Glu Gly Thr Ile Ser Tyr Pro Leu
                325                 330                 335

Glu Glu Thr Lys Ile Thr Arg Tyr Phe Pro Asp Glu Thr Ile Asn Ala
            340                 345                 350

Cys Lys Thr Met Lys Ser Arg Ser Phe Val Tyr Ser Arg Gly Arg Lys
        355                 360                 365

Leu Val Gly Gly Val Asn Gln Asp Val Glu Tyr Ser Ser Ile Thr Asp
    370                 375                 380

Gln Gln Leu Thr Thr Glu Trp Gln Cys Gln Val Gln Lys Ile Thr Arg
385                 390                 395                 400

Ser His Ser Thr Asp Ile Pro Tyr Ile Val Ser Glu Ala Ala Val Gln
                405                 410                 415

Ala Glu Gln Lys Glu Gln Phe Ala Asp Met Gln Asp Glu His His Val
            420                 425                 430

Ala Glu Ala Ile Pro Arg Ile Pro Arg Leu Ser Leu Thr Ile Thr Asp
        435                 440                 445

Arg Asn Gly Met Glu Asn Leu Leu Ser Val Lys Pro Asp Gln Thr Leu
    450                 455                 460

Gly Phe Pro Ser Leu Arg Ser Lys Ser Leu His Gly His Pro Arg Asn
465                 470                 475                 480

Val Lys Ser Ile Gln Gly Lys Leu Asp Arg Ser Gly His Ala Ser Ser
                485                 490                 495

Val Ser Ser Leu Val Ile Val Ser Gly Met Thr Ala Glu Glu Lys Lys
            500                 505                 510

Val Lys Lys Glu Lys Ala Ser Thr Glu Thr Glu Cys
        515                 520


<210> 6
<211> 17
<212> PRT
<213> Homo sapiens

<400> 6
Met Ser Glu Pro Gly Lys Leu Ser Gln Lys Ile Lys Val Trp Leu Gln
1               5                   10                  15

Glu


<210> 7
<211> 21

<212> PRT
<213> Homo sapiens

<400> 7
Ile Leu Met Ser Glu Pro Gly Lys Leu Ser Gln Lys Ile Lys Val Trp
1               5                   10                  15

Leu Gln Glu Tyr Trp
            20


<210> 8
<211> 13
<212> PRT
<213> Homo sapiens

<400> 8
Asn Lys Tyr Leu Gly Pro Tyr Val Met Met Ile Gly Lys
1               5                   10


<210> 9
<211> 17
<212> PRT
<213> Homo sapiens

<400> 9
Gly Val Asn Lys Tyr Leu Gly Pro Tyr Val Met Met Ile Gly Lys Met
1               5                   10                  15

Met


<210> 10
<211> 8
<212> PRT
<213> Homo sapiens

<400> 10
Arg Met Asp Gly Trp Pro Ser Leu
1               5


<210> 11
<211> 12
<212> PRT
<213> Homo sapiens

<400> 11
Leu Val Arg Met Asp Gly Trp Pro Ser Leu Gln Glu
1               5                   10


<210> 12
<211> 9
<212> PRT
<213> Homo sapiens

<400> 12

Asn Gln Pro Tyr Met Gly Tyr Gly Arg
1               5

<210> 13
<211> 13
<212> PRT
<213> Homo sapiens

<400> 13
Leu Gln Asn Gln Pro Tyr Met Gly Tyr Gly Arg Val Ile
1               5                   10

<210> 14
<211> 72
<212> PRT
<213> Homo sapiens

<400> 14
Ala Arg Gln Ala Ile Leu His Pro Glu Glu Lys Pro Ser Trp Lys Leu
1               5                   10                  15

Ala Arg Asn Ile Phe Tyr Met Pro Tyr Trp Met Ile Tyr Gly Glu Val
            20                  25                  30

Phe Ala Asp Gln Ile Asp Leu Tyr Ala Met Glu Ile Asn Pro Pro Cys
        35                  40                  45

Gly Glu Asn Leu Tyr Asp Glu Glu Gly Lys Arg Leu Pro Pro Cys Ile
    50                  55                  60

Pro Gly Ala Trp Leu Thr Pro Ala
65                  70

<210> 15
<211> 76
<212> PRT
<213> Homo sapiens

<400> 15
Gly Val Ala Arg Gln Ala Ile Leu His Pro Glu Glu Lys Pro Ser Trp
1               5                   10                  15

Lys Leu Ala Arg Asn Ile Phe Tyr Met Pro Tyr Trp Met Ile Tyr Gly
            20                  25                  30

Glu Val Phe Ala Asp Gln Ile Asp Leu Tyr Ala Met Glu Ile Asn Pro
        35                  40                  45

Pro Cys Gly Glu Asn Leu Tyr Asp Glu Glu Gly Lys Arg Leu Pro Pro
    50                  55                  60

Cys Ile Pro Gly Ala Trp Leu Thr Pro Ala Leu Met
65                  70                  75

<210> 16
<211> 19

<212> PRT
<213> Homo sapiens

<400> 16
Pro Glu Glu Lys Pro Ser Trp Lys Leu Ala Arg Asn Ile Phe Tyr Met
1               5                   10                  15

Pro Tyr Trp

<210> 17
<211> 15
<212> PRT
<213> Homo sapiens

<400> 17
Asn Pro Pro Cys Gly Glu Asn Leu Tyr Asp Glu Glu Gly Lys Arg
1               5                   10                  15

<210> 18
<211> 259
<212> PRT
<213> Homo sapiens

<400> 18
Pro Ile Val Lys Phe Trp Phe Tyr Thr Ile Ser Tyr Leu Gly Tyr Leu
1               5                   10                  15

Leu Leu Phe Asn Tyr Val Ile Leu Val Arg Met Asp Gly Trp Pro Ser
            20                  25                  30

Leu Gln Glu Trp Ile Val Ile Ser Tyr Ile Val Ser Leu Ala Leu Glu
        35                  40                  45

Lys Ile Arg Glu Ile Leu Met Ser Glu Pro Gly Lys Leu Ser Gln Lys
    50                  55                  60

Ile Lys Val Trp Leu Gln Glu Tyr Trp Asn Ile Thr Asp Leu Val Ala
65                  70                  75                  80

Ile Ser Thr Phe Met Ile Gly Ala Ile Leu Arg Leu Gln Asn Gln Pro
                85                  90                  95

Tyr Met Gly Tyr Gly Arg Val Ile Tyr Cys Val Asp Ile Ile Phe Trp
            100                 105                 110

Tyr Ile Arg Val Leu Asp Ile Phe Gly Val Asn Lys Tyr Leu Gly Pro
        115                 120                 125

Tyr Val Met Met Ile Gly Lys Met Met Ile Asp Met Leu Tyr Phe Val
    130                 135                 140

Val Ile Met Leu Val Val Leu Met Ser Phe Gly Val Ala Arg Gln Ala
145                 150                 155                 160

Ile Leu His Pro Glu Glu Lys Pro Ser Trp Lys Leu Ala Arg Asn Ile
                165                 170                 175

Phe Tyr Met Pro Tyr Trp Met Ile Tyr Gly Glu Val Phe Ala Asp Gln
            180                 185                 190

Ile Asp Leu Tyr Ala Met Glu Ile Asn Pro Pro Cys Gly Glu Asn Leu
        195                 200                 205

Tyr Asp Glu Glu Gly Lys Arg Leu Pro Pro Cys Ile Pro Gly Ala Trp
    210                 215                 220

Leu Thr Pro Ala Leu Met Ala Cys Tyr Leu Leu Val Ala Asn Ile Leu
225                 230                 235                 240

Leu Val Asn Leu Leu Ile Ala Val Phe Asn Asn Thr Phe Phe Glu Val
                245                 250                 255

Lys Ser Ile

<210> 19
<211> 263
<212> PRT
<213> Homo sapiens

<400> 19
Asn Ala Pro Ile Val Lys Phe Trp Phe Tyr Thr Ile Ser Tyr Leu Gly
  1               5                  10                  15

Tyr Leu Leu Leu Phe Asn Tyr Val Ile Leu Val Arg Met Asp Gly Trp
             20                  25                  30

Pro Ser Leu Gln Glu Trp Ile Val Ile Ser Tyr Ile Val Ser Leu Ala
         35                  40                  45

Leu Glu Lys Ile Arg Glu Ile Leu Met Ser Glu Pro Gly Lys Leu Ser
     50                  55                  60

Gln Lys Ile Lys Val Trp Leu Gln Glu Tyr Trp Asn Ile Thr Asp Leu
 65                  70                  75                  80

Val Ala Ile Ser Thr Phe Met Ile Gly Ala Ile Leu Arg Leu Gln Asn
                 85                  90                  95

Gln Pro Tyr Met Gly Tyr Gly Arg Val Ile Tyr Cys Val Asp Ile Ile
            100                 105                 110

Phe Trp Tyr Ile Arg Val Leu Asp Ile Phe Gly Val Asn Lys Tyr Leu
        115                 120                 125

Gly Pro Tyr Val Met Met Ile Gly Lys Met Met Ile Asp Met Leu Tyr
    130                 135                 140

Phe Val Val Ile Met Leu Val Val Leu Met Ser Phe Gly Val Ala Arg
145                 150                 155                 160

Gln Ala Ile Leu His Pro Glu Glu Lys Pro Ser Trp Lys Leu Ala Arg
                165                 170                 175

Asn Ile Phe Tyr Met Pro Tyr Trp Met Ile Tyr Gly Glu Val Phe Ala
            180                 185                 190

Asp Gln Ile Asp Leu Tyr Ala Met Glu Ile Asn Pro Pro Cys Gly Glu
        195                 200                 205

Asn Leu Tyr Asp Glu Glu Gly Lys Arg Leu Pro Pro Cys Ile Pro Gly
    210                 215                 220

Ala Trp Leu Thr Pro Ala Leu Met Ala Cys Tyr Leu Leu Val Ala Asn
225                 230                 235                 240

Ile Leu Leu Val Asn Leu Leu Ile Ala Val Phe Asn Asn Thr Phe Phe
                245                 250                 255

Glu Val Lys Ser Ile Ser Asn
            260


<210> 20
<211> 63
<212> PRT
<213> Homo sapiens

<400> 20
His Pro Glu Glu Lys Pro Ser Trp Lys Leu Ala Arg Asn Ile Phe Tyr
  1               5                  10                  15

Met Pro Tyr Trp Met Ile Tyr Gly Glu Val Phe Ala Asp Gln Ile Asp
             20                  25                  30

Leu Tyr Ala Met Glu Ile Asn Pro Pro Cys Gly Glu Asn Leu Tyr Asp
         35                  40                  45

Glu Glu Gly Lys Arg Leu Pro Pro Cys Ile Pro Gly Ala Trp Leu
     50                  55                  60


<210> 21
<211> 23
<212> PRT
<213> Homo sapiens

<400> 21
Glu Lys Pro Ser Trp Lys Leu Ala Arg Asn Ile Phe Tyr Met Pro Tyr
  1               5                  10                  15

Trp Met Ile Tyr Gly Glu Val
             20


<210> 22
<211> 28
<212> PRT
<213> Homo sapiens

<400> 22
Asn Pro Pro Cys Gly Glu Asn Leu Tyr Asp Glu Glu Gly Lys Arg Leu
  1               5                  10                  15

Pro Pro Cys Ile Pro Gly Ala Trp Leu Thr Pro Ala
             20                  25

<210> 23
<211> 4602
<212> DNA
<213> Homo sapiens

<400> 23
atgtatatcc gtgtatccta tgacaccaag ccagactcac tgctccatct catggtgaaa 60
gattggcagc tggaactccc caagctctta atatctgtgc atggaggcct ccagaacttt 120
gagatgcagc ccaagctgaa acaagtcttt gggaaaggcc tgatcaaggc tgctatgacc 180
accggggcct ggatcttcac cgggggtgtc agcacaggtg ttatcagcca cgtaggggat 240
gccttgaaag accactcctc caagtccaga ggccgggttt gtgctatagg aattgctcca 300
tggggcatcg tggagaataa ggaagacctg gttggaaagg atgtaacaag agtgtaccag 360
accatgtcca accctctaag taagctctct gtgctcaaca actcccacac ccacttcatc 420
ctggctgaca atggcaccct gggcaagtat ggcgccgagg tgaagctgcg aaggctgctg 480
gaaaagcaca tctccctcca gaagatcaac acaagactgg ggcagggcgt gcccctcgtg 540
ggtctcgtgg tggagggggg ccctaacgtg gtgtccatcg tcttggaata cctgcaagaa 600
gagcctccca tccctgtggt gatttgtgat ggcagcggac gtgcctcgga catcctgtcc 660
tttgcgcaca agtactgtga agaaggcgga ataataaatg agtccctcag ggagcagctt 720
ctagttacca ttcagaaaac atttaattat aataaggcac aatcacatca gctgtttgca 780
attataatgg agtgcatgaa gaagaaagaa ctcgtcactg tgttcagaat gggttctgag 840
ggccagcagg acatcgagat ggcaatttta actgccctgc tgaaaggaac aaacgtatct 900
gctccagatc agctgagctt ggcactggct tggaaccgcg tggacatagc acgaagccag 960
atctttgtct ttgggcccca ctggacgccc ctgggaagcc tggcaccccc gacggacagc 1020
aaagccacgg agaaggagaa gaagccaccc atggccacca ccaagggagg aagaggaaaa 1080
gggaaaggca agaagaaagg gaaagtgaaa gaggaagtgg aggaagaaac tgacccccgg 1140
aagatagagc tgctgaactg ggtgaatgct ttggagcaag cgatgctaga tgctttagtc 1200
ttagatcgtg tcgactttgt gaagctcctg attgaaaacg gagtgaacat gcaacacttt 1260
ctgaccattc cgaggctgga ggagctctat aacacaagac tgggtccacc aaacacactt 1320
catctgctgg tgagggatgt gaaaaagagc aaccttccgc ctgattacca catcagcctc 1380
atagacatcg ggctcgtgct ggagtacctc atgggaggag cctaccgctg caactacact 1440
cggaaaaact ttcggaccct ttacaacaac ttgtttggac caaagaggcc taaagctctt 1500
aaacttctgg gaatggaaga tgatgagcct ccagctaaag ggaagaaaaa aaaaaaaaag 1560
aaaaaggagg aagagatcga cattgatgtg gacgaccctg ccgtgagtcg gttccagtat 1620
cccttccacg agctgatggt gtgggcagtg ctgatgaaac gccagaaaat ggcagtgttc 1680
ctctggcagc gaggggaaga gagcatggcc aaggccctgg tggcctgcaa gctctacaag 1740
gccatggccc acgagtcctc cgagagtgat ctggtggatg acatctccca ggacttggat 1800
aacaattcca aagacttcgg ccagcttgct ttggagttat tagaccagtc ctataagcat 1860
gacgagcaga tcgctatgaa actcctgacc tacgagctga aaaactggag caactcgacc 1920
tgcctcaaac tggccgtggc agccaaacac cgggacttca ttgctcacac ctgcagccag 1980
atgctgctga ccgatatgtg gatgggaaga ctgcggatgc ggaagaaccc cggcctgaag 2040
gttatcatgg ggattcttct accccccacc atcttgtttt tggaatttcg cacatatgat 2100
gatttctcgt atcaaacatc caaggaaaac gaggatggca aagaaaaaga agaggaaaat 2160
acggatgcaa atgcagatgc tggctcaaga aagggggatg aggagaacga gcataaaaaa 2220
cagagaagta ttcccatcgg aacaaagatc tgtgaattct ataacgcgcc cattgtcaag 2280
ttctggtttt acacaatatc atacttgggc tacctgctgc tgtttaacta cgtcatcctg 2340
gtgcggatgg atggctggcc gtccctccag gagtggatcg tcatctccta catcgtgagc 2400
ctggcgttag agaagatacg agagatcctc atgtcagaac caggcaaact cagccagaaa 2460
atcaaagttt ggcttcagga gtactggaac atcacagatc tcgtggccat ttccacattc 2520
atgattggag caattcttcg cctacagaac cagccctaca tgggctatgg ccgggtgatc 2580
tactgtgtgg atatcatctt ctggtacatc cgtgtcctgg acatctttgg tgtcaacaag 2640
tatctggggc catacgtgat gatgattgga aagatgatga tcgacatgct gtactttgtg 2700
gtcatcatgc tggtcgtgct catgagtttc ggagtagccc gtcaagccat tctgcatcca 2760
gaggagaagc cctcttggaa actggcccga aacatcttct acatgcccta ctggatgatc 2820
tatggagagg tgtttgcaga ccagatagac ctctacgcca tggaaattaa tcctccttgt 2880
ggtgagaacc tatatgatga ggagggcaag cggcttcctc cctgtatccc cggcgcctgg 2940
ctcactccag cactcatggc gtgctatcta ctggtcgcca acatcctgct ggtgaacctg 3000
ctgattgctg tgttcaacaa tactttcttt gaagtaaaat caatatccaa ccaggtgtgg 3060
aagttccagc gatatcagct gattatgaca tttcatgaca ggccagtcct gcccccaccg 3120

atgatcattt taagccacat ctacatcatc attatgcgtc tcagcggccg ctgcaggaaa 3180
aagagagaag gggaccaaga ggaacgggat cgtggattga agctcttcct tagcgacgag 3240
gagctaaaga ggctgcatga gttcgaggag cagtgcgtgc aggagcactt ccgggagaag 3300
gaggatgagc agcagtcgtc cagcgacgag cgcatccggg tcacttctga aagagttgaa 3360
aatatgtcaa tgaggttgga agaaatcaat gaaagagaaa cttttatgaa aacttccctg 3420
cagactgttg accttcgact tgctcagcta gaagaattat ctaacagaat ggtgaatgct 3480
cttgaaaatc ttgcgggaat cgacaggtct gacctgatcc aggcacggtc ccgggcttct 3540
tctgaatgtg aggcaacgta tcttctccgg caaagcagca tcaatagcgc tgatggctac 3600
agcttgtatc gatatcattt taacggagaa gagttattat ttgaggatac atctctctcc 3660
acgtcaccag ggacaggagt caggaaaaaa acctgttcct tccgtataaa ggaagagaag 3720
gacgtgaaaa cgcacctagt cccagaatgt cagaacagtc ttcacctttc actgggcaca 3780
agcacatcag caaccccaga tggcagtcac cttgcagtag atgacttaaa gaacgctgaa 3840
gagtcaaaat taggtccaga tattgggatt tcaaaggaag atgatgaaag acagacagac 3900
tctaaaaaag aagaaactat ttccccaagt ttaaataaaa cagatgtgat acatggacag 3960
gacaaatcag atgttcaaaa cactcagcta acagtggaaa cgacaaatat agaaggcact 4020
atttcctatc ccctggaaga aaccaaaatt acacgctatt tccccgatga aacgatcaat 4080
gcttgtaaaa caatgaagtc cagaagcttc gtctattccc ggggaagaaa gctggtcggt 4140
ggggttaacc aggatgtaga gtacagttca atcacggacc agcaattgac gacggaatgg 4200
caatgccaag ttcaaaagat cacgcgctct catagcacag atattcctta cattgtgtcg 4260
gaagctgcag tgcaagctga gcaaaaagag cagtttgcag atatgcaaga tgaacaccat 4320
gtcgctgaag caattcctcg aatccctcgc ttgtccctaa ccattactga cagaaatggg 4380
atggaaaact tactgtctgt gaagccagat caaactttgg gattcccatc tctcaggtca 4440
aaaagtttac atggacatcc taggaatgtg aaatccattc agggaaagtt agacagatct 4500
ggacatgcca gtagtgtaag cagcttagta attgtgtctg gaatgacagc agaagaaaaa 4560
aaggttaaga aagagaaagc ttccacagaa actgaatgct ag 4602

<210> 24
<211> 2268
<212> DNA
<213> Homo sapiens

<400> 24
atgtatatcc gtgtatccta tgacaccaag ccagactcac tgctccatct catggtgaaa 60
gattggcagc tggaactccc caagctctta atatctgtgc atggaggcct ccagaacttt 120
gagatgcagc ccaagctgaa acaagtcttt gggaaaggcc tgatcaaggc tgctatgacc 180
accggggcct ggatcttcac cgggggtgtc agcacaggtg ttatcagcca cgtaggggat 240
gccttgaaag accactcctc caagtccaga ggccgggttt gtgctatagg aattgctcca 300
tggggcatcg tggagaataa ggaagacctg gttggaaagg atgtaacaag agtgtaccag 360
accatgtcca accctctaag taagctctct gtgctcaaca actcccacac ccacttcatc 420
ctggctgaca atggcaccct gggcaagtat ggcgccgagg tgaagctgcg aaggctgctg 480
gaaaagcaca tctccctcca gaagatcaac acaagactgg ggcagggcgt gcccctcgtg 540
ggtctcgtgg tggagggggg ccctaacgtg gtgtccatcg tcttggaata cctgcaagaa 600
gagcctccca tccctgtggt gatttgtgat ggcagcggac gtgcctcgga catcctgtcc 660
tttgcgcaca agtactgtga agaaggcgga ataataaatg agtccctcag ggagcagctt 720
ctagttacca ttcagaaaac atttaattat aataaggcac aatcacatca gctgtttgca 780
attataatgg agtgcatgaa gaagaaagaa ctcgtcactg tgttcagaat gggttctgag 840
ggccagcagg acatcgagat ggcaatttta actgccctgc tgaaaggaac aaacgtatct 900
gctccagatc agctgagctt ggcactggct tggaaccgcg tggacatagc acgaagccag 960
atctttgtct ttgggcccca ctggacgccc ctgggaagcc tggcaccccc gacggacagc 1020
aaagccacgg agaaggagaa gaagccaccc atggccacca ccaagggagg aagaggaaaa 1080
gggaaaggca agaagaaagg gaaagtgaaa gaggaagtgg aggaagaaac tgacccccgg 1140
aagatagagc tgctgaactg ggtgaatgct ttggagcaag cgatgctaga tgctttagtc 1200
ttagatcgtg tcgactttgt gaagctcctg attgaaaacg gagtgaacat gcaacacttt 1260
ctgaccattc cgaggctgga ggagctctat aacacaagac tgggtccacc aaacacactt 1320
catctgctgg tgagggatgt gaaaaagagc aaccttccgc ctgattacca catcagcctc 1380
atagacatcg ggctcgtgct ggagtacctc atgggaggag cctaccgctg caactacact 1440
cggaaaaact ttcggaccct ttacaacaac ttgtttggac caaagaggcc taaagctctt 1500
aaacttctgg gaatggaaga tgatgagcct ccagctaaag ggaagaaaaa aaaaaaaaag 1560
aaaaaggagg aagagatcga cattgatgtg gacgaccctg ccgtgagtcg gttccagtat 1620

cccttccacg agctgatggt gtgggcagtg ctgatgaaac gccagaaaat ggcagtgttc 1680
ctctggcagc gaggggaaga gagcatggcc aaggccctgg tggcctgcaa gctctacaag 1740
gccatggccc acgagtcctc cgagagtgat ctggtggatg acatctccca ggacttggat 1800
aacaattcca aagacttcgg ccagcttgct ttggagttat tagaccagtc ctataagcat 1860
gacgagcaga tcgctatgaa actcctgacc tacgagctga aaaactggag caactcgacc 1920
tgcctcaaac tggccgtggc agccaaacac cgggacttca ttgctcacac ctgcagccag 1980
atgctgctga ccgatatgtg gatgggaaga ctgcggatgc ggaagaaccc cggcctgaag 2040
gttatcatgg ggattcttct accccccacc atcttgtttt tggaatttcg cacatatgat 2100
gatttctcgt atcaaacatc caaggaaaac gaggatggca aagaaaaaga agaggaaaat 2160
acggatgcaa atgcagatgc tggctcaaga aagggggatg aggagaacga gcataaaaaa 2220
cagagaagta ttcccatcgg aacaaagatc tgtgaattct ataacgcg 2268

<210> 25
<211> 2283
<212> DNA
<213> Homo sapiens

<400> 25
atgtatatcc gtgtatccta tgacaccaag ccagactcac tgctccatct catggtgaaa 60
gattggcagc tggaactccc caagctctta atatctgtgc atggaggcct ccagaacttt 120
gagatgcagc ccaagctgaa acaagtcttt gggaaaggcc tgatcaaggc tgctatgacc 180
accggggcct ggatcttcac cgggggtgtc agcacaggtg ttatcagcca cgtaggggat 240
gccttgaaag accactcctc caagtccaga ggccgggttt gtgctatagg aattgctcca 300
tggggcatcg tggagaataa ggaagacctg gttggaaagg atgtaacaag agtgtaccag 360
accatgtcca accctctaag taagctctct gtgctcaaca actcccacac ccacttcatc 420
ctggctgaca atggcaccct gggcaagtat ggcgccgagg tgaagctgcg aaggctgctg 480
gaaaagcaca tctccctcca gaagatcaac acaagactgg ggcagggcgt gcccctcgtg 540
ggtctcgtgg tggagggggg ccctaacgtg gtgtccatcg tcttggaata cctgcaagaa 600
gagcctccca tccctgtggt gatttgtgat ggcagcggac gtgcctcgga catcctgtcc 660
tttgcgcaca agtactgtga agaaggcgga ataataaatg agtccctcag ggagcagctt 720
ctagttacca ttcagaaaac atttaattat aataaggcac aatcacatca gctgtttgca 780
attataatgg agtgcatgaa gaagaaagaa ctcgtcactg tgttcagaat gggttctgag 840
ggccagcagg acatcgagat ggcaatttta actgccctgc tgaaaggaac aaacgtatct 900
gctccagatc agctgagctt ggcactggct tggaaccgcg tggacatagc acgaagccag 960
atctttgtct ttggccccca ctggacgccc ctgggaagcc tggcaccccc gacggacagc 1020
aaagccacgg agaaggagaa gaagccaccc atggccacca ccaagggagg aagaggaaaa 1080
gggaaaggca agaagaaagg gaaagtgaaa gaggaagtgg aggaagaaac tgacccccgg 1140
aagatagagc tgctgaactg ggtgaatgct ttggagcaag cgatgctaga tgctttagtc 1200
ttagatcgtg tcgactttgt gaagctcctg attgaaaacg gagtgaacat gcaacacttt 1260
ctgaccattc cgaggctgga ggagctctat aacacaagac tgggtccacc aaacacactt 1320
catctgctgg tgagggatgt gaaaaagagc aaccttccgc ctgattacca catcagcctc 1380
atagacatcg ggctcgtgct ggagtacctc atgggaggag cctaccgctg caactacact 1440
cggaaaaact ttcggaccct ttacaacaac ttgtttggac caaagaggcc taaagctctt 1500
aaacttctgg gaatggaaga tgatgagcct ccagctaaag ggaagaaaaa aaaaaaaaag 1560
aaaaaggagg aagagatcga cattgatgtg gacgaccctg ccgtgagtcg gttccagtat 1620
cccttccacg agctgatggt gtgggcagtg ctgatgaaac gccagaaaat ggcagtgttc 1680
ctctggcagc gaggggaaga gagcatggcc aaggccctgg tggcctgcaa gctctacaag 1740
gccatggccc acgagtcctc cgagagtgat ctggtggatg acatctccca ggacttggat 1800
aacaattcca aagacttcgg ccagcttgct ttggagttat tagaccagtc ctataagcat 1860
gacgagcaga tcgctatgaa actcctgacc tacgagctga aaaactggag caactcgacc 1920
tgcctcaaac tggccgtggc agccaaacac cgggacttca ttgctcacac ctgcagccag 1980
atgctgctga ccgatatgtg gatgggaaga ctgcggatgc ggaagaaccc cggcctgaag 2040
gttatcatgg ggattcttct accccccacc atcttgtttt tggaatttcg cacatatgat 2100
gatttctcgt atcaaacatc caaggaaaac gaggatggca aagaaaaaga agaggaaaat 2160
acggatgcaa atgcagatgc tggctcaaga aagggggatg aggagaacga gcataaaaaa 2220
cagagaagta ttcccatcgg aacaaagatc tgtgaattct ataacgcgcc cattgtcaag 2280
ttc 2283

<210> 26
<211> 1554
<212> DNA
<213> Homo sapiens

<400> 26
tccaaccagg tgtggaagtt ccagcgatat cagctgatta tgacatttca tgacaggcca 60
gtcctgcccc caccgatgat cattttaagc cacatctaca tcatcattat gcgtctcagc 120
ggccgctgca ggaaaaagag agaaggggac caagaggaac gggatcgtgg attgaagctc 180
ttccttagcg acgaggagct aaagaggctg catgagttcg aggagcagtg cgtgcaggag 240
cacttccggg agaaggagga tgagcagcag tcgtccagcg acgagcgcat ccgggtcact 300
tctgaaagag ttgaaaatat gtcaatgagg ttggaagaaa tcaatgaaag agaaactttt 360
atgaaaactt ccctgcagac tgttgacctt cgacttgctc agctagaaga attatctaac 420
agaatggtga atgctcttga aaatcttgcg ggaatcgaca ggtctgacct gatccaggca 480
cggtcccggg cttcttctga atgtgaggca acgtatcttc tccggcaaag cagcatcaat 540
agcgctgatg gctacagctt gtatcgatat cattttaacg gagaagagtt attatttgag 600
gatacatctc tctccacgtc accagggaca ggagtcagga aaaaaacctg ttccttccgt 660
ataaaggaag agaaggacgt gaaaacgcac ctagtcccag aatgtcagaa cagtcttcac 720
ctttcactgg gcacaagcac atcagcaacc ccagatggca gtcaccttgc agtagatgac 780
ttaaagaacg ctgaagagtc aaaattaggt ccagatattg ggatttcaaa ggaagatgat 840
gaaagacaga cagactctaa aaaagaagaa actatttccc caagtttaaa taaaacagat 900
gtgatacatg gacaggacaa atcagatgtt caaaacactc agctaacagt ggaaacgaca 960
aatatagaag gcactatttc ctatcccctg gaagaaacca aaattacacg ctatttcccc 1020
gatgaaacga tcaatgcttg taaaacaatg aagtccagaa gcttcgtcta ttcccgggga 1080
agaaagctgg tcggtggggt taaccaggat gtagagtaca gttcaatcac ggaccagcaa 1140
ttgacgacgg aatggcaatg ccaagttcaa aagatcacgc gctctcatag cacagatatt 1200
ccttacattg tgtcggaagc tgcagtgcaa gctgagcaaa aagagcagtt tgcagatatg 1260
caagatgaac accatgtcgc tgaagcaatt cctcgaatcc ctcgcttgtc cctaaccatt 1320
actgacagaa atgggatgga aaacttactg tctgtgaagc cagatcaaac tttgggattc 1380
ccatctctca ggtcaaaaag tttacatgga catcctagga atgtgaaatc cattcaggga 1440
aagttagaca gatctggaca tgccagtagt gtaagcagct tagtaattgt gtctggaatg 1500
acagcagaag aaaaaaaggt taagaaagag aaagcttcca cagaaactga atgc 1554

<210> 27
<211> 1575
<212> DNA
<213> Homo sapiens

<400> 27
tttgaagtaa aatcaatatc caaccaggtg tggaagttcc agcgatatca gctgattatg 60
acatttcatg acaggccagt cctgccccca ccgatgatca ttttaagcca catctacatc 120
atcattatgc gtctcagcgg ccgctgcagg aaaaagagag aaggggacca agaggaacgg 180
gatcgtggat tgaagctctt ccttagcgac gaggagctaa agaggctgca tgagttcgag 240
gagcagtgcg tgcaggagca cttccgggag aaggaggatg agcagcagtc gtccagcgac 300
gagcgcatcc gggtcacttc tgaaagagtt gaaaatatgt caatgaggtt ggaagaaatc 360
aatgaaagag aaacttttat gaaaacttcc ctgcagactg ttgaccttcg acttgctcag 420
ctagaagaat tatctaacag aatggtgaat gctcttgaaa atcttgcggg aatcgacagg 480
tctgacctga tccaggcacg gtcccgggct tcttctgaat gtgaggcaac gtatcttctc 540
cggcaaagca gcatcaatag cgctgatggc tacagcttgt atcgatatca ttttaacgga 600
gaagagttat tatttgagga tacatctctc tccacgtcac cagggacagg agtcaggaaa 660
aaaacctgtt ccttccgtat aaaggaagag aaggacgtga aaacgcacct agtcccagaa 720
tgtcagaaca gtcttcacct ttcactgggc acaagcacat cagcaacccc agatggcagt 780
caccttgcag tagatgactt aaagaacgct gaagagtcaa aattaggtcc agatattggg 840
atttcaaagg aagatgatga aagacagaca gactctaaaa aagaagaaac tatttcccca 900
agtttaaata aaacagatgt gatacatgga caggacaaat cagatgttca aaacactcag 960
ctaacagtgg aaacgacaaa tatagaaggc actatttcct atcccctgga agaaaccaaa 1020
attacacgct atttccccga tgaaacgatc aatgcttgta aaacaatgaa gtccagaagc 1080
ttcgtctatt cccggggaag aaagctggtc ggtggggtta accaggatgt agagtacagt 1140
tcaatcacgg accagcaatt gacgacggaa tggcaatgcc aagttcaaaa gatcacgcgc 1200

tctcatagca cagatattcc ttacattgtg tcggaagctg cagtgcaagc tgagcaaaaa 1260
gagcagtttg cagatatgca agatgaacac catgtcgctg aagcaattcc tcgaatccct 1320
cgcttgtccc taaccattac tgacagaaat gggatggaaa acttactgtc tgtgaagcca 1380
gatcaaactt tgggattccc atctctcagg tcaaaaagtt tacatggaca tcctaggaat 1440
gtgaaatcca ttcagggaaa gttagacaga tctggacatg ccagtagtgt aagcagctta 1500
gtaattgtgt ctggaatgac agcagaagaa aaaaaggtta agaaagagaa agcttccaca 1560
gaaactgaat gctag 1575

<210> 28
<211> 51
<212> DNA
<213> Homo sapiens

<400> 28
atgtcagaac caggcaaact cagccagaaa atcaaagttt ggcttcagga g 51

<210> 29
<211> 63
<212> DNA
<213> Homo sapiens

<400> 29
atcctcatgt cagaaccagg caaactcagc cagaaaatca aagtttggct tcaggagtac 60
tgg 63

<210> 30
<211> 39
<212> DNA
<213> Homo sapiens

<400> 30
aacaagtatc tggggccata cgtgatgatg attggaaag 39

<210> 31
<211> 51
<212> DNA
<213> Homo sapiens

<400> 31
ggtgtcaaca agtatctggg gccatacgtg atgatgattg gaaagatgat g 51

<210> 32
<211> 24
<212> DNA
<213> Homo sapiens

<400> 32
cggatggatg gctggccgtc cctc 24

<210> 33
<211> 36
<212> DNA
<213> Homo sapiens

<400> 33
ctggtgcgga tggatggctg gccgtccctc caggag                                36

<210> 34
<211> 27
<212> DNA
<213> Homo sapiens

<400> 34
aaccagccct acatgggcta tggccgg                                          27

<210> 35
<211> 39
<212> DNA
<213> Homo sapiens

<400> 35
ctacagaacc agccctacat gggctatggc cgggtgatc                             39

<210> 36
<211> 216
<212> DNA
<213> Homo sapiens

<400> 36
gcccgtcaag ccattctgca tccagaggag aagccctctt ggaaactggc ccgaaacatc 60
ttctacatgc cctactggat gatctatgga gaggtgtttg cagaccagat agacctctac 120
gccatggaaa ttaatcctcc ttgtggtgag aacctatatg atgaggaggg caagcggctt 180
cctccctgta tccccggcgc ctggctcact ccagca                               216

<210> 37
<211> 228
<212> DNA
<213> Homo sapiens

<400> 37
ggagtagccc gtcaagccat tctgcatcca gaggagaagc cctcttggaa actggcccga 60
aacatcttct acatgcccta ctggatgatc tatggagagg tgtttgcaga ccagatagac 120
ctctacgcca tggaaattaa tcctccttgt ggtgagaacc tatatgatga ggagggcaag 180
cggcttcctc cctgtatccc cggcgcctgg ctcactccag cactcatg                   228

<210> 38
<211> 57
<212> DNA
<213> Homo sapiens

<400> 38
ccagaggaga agccctcttg gaaactggcc cgaaacatct tctacatgcc ctactgg    57

<210> 39
<211> 777
<212> DNA
<213> Homo sapiens

<400> 39
cccattgtca agttctggtt ttacacaata tcatacttgg gctacctgct gctgtttaac 60
tacgtcatcc tggtgcggat ggatggctgg ccgtccctcc aggagtggat cgtcatctcc 120
tacatcgtga gcctggcgtt agagaagata cgagagatcc tcatgtcaga accaggcaaa 180
ctcagccaga aaatcaaagt ttggcttcag gagtactgga acatcacaga tctcgtggcc 240
atttccacat tcatgattgg agcaattctt cgcctacaga accagcccta catgggctat 300
ggccgggtga tctactgtgt ggatatcatc ttctggtaca tccgtgtcct ggacatcttt 360
ggtgtcaaca agtatctggg gccatacgtg atgatgattg gaaagatgat gatcgacatg 420
ctgtactttg tggtcatcat gctggtcgtg ctcatgagtt tcggagtagc ccgtcaagcc 480
attctgcatc cagaggagaa gccctcttgg aaactggccc gaaacatctt ctacatgccc 540
tactggatga tctatggaga ggtgtttgca gaccagatag acctctacgc catggaaatt 600
aatcctcctt gtggtgagaa cctatatgat gaggagggca agcggcttcc tccctgtatc 660
cccggcgcct ggctcactcc agcactcatg gcgtgctatc tactggtcgc caacatcctg 720
ctggtgaacc tgctgattgc tgtgttcaac aatactttct ttgaagtaaa atcaata 777

<210> 40
<211> 789
<212> DNA
<213> Homo sapiens

<400> 40
aacgcgccca ttgtcaagtt ctggttttac acaatatcat acttgggcta cctgctgctg 60
tttaactacg tcatcctggt gcggatggat ggctggccgt ccctccagga gtggatcgtc 120
atctcctaca tcgtgagcct ggcgttagag aagatacgag agatcctcat gtcagaacca 180
ggcaaactca gccagaaaat caaagtttgg cttcaggagt actggaacat cacagatctc 240
gtggccattt ccacattcat gattggagca attcttcgcc tacagaacca gccctacatg 300
ggctatggcc gggtgatcta ctgtgtggat atcatcttct ggtacatccg tgtcctggac 360
atctttggtg tcaacaagta tctggggcca tacgtgatga tgattggaaa gatgatgatc 420
gacatgctgt actttgtggt catcatgctg gtcgtgctca tgagtttcgg agtagcccgt 480
caagccattc tgcatccaga ggagaagccc tcttggaaac tggcccgaaa catcttctac 540
atgccctact ggatgatcta tggagaggtg tttgcagacc agatagacct ctacgccatg 600
gaaattaatc ctccttgtgg tgagaaccta tatgatgagg agggcaagcg gcttcctccc 660
tgtatccccg gcgcctggct cactccagca ctcatggcgt gctatctact ggtcgccaac 720
atcctgctgg tgaacctgct gattgctgtg ttcaacaata ctttctttga agtaaaatca 780
atatccaac 789

<210> 41
<211> 50
<212> DNA
<213> Artificial Sequence

<220>
<223> Description of Artificial Sequence:promoter

<400> 41
ttacagaaaa tataagtggc ggggagtcat tgcccaactc attttatgag 50

<210> 42
<211> 50
<212> DNA
<213> Artificial Sequence

<220>
<223> Description of Artificial Sequence:promoter

<400> 42
attaaaaata caaaaaaatt ggccgggcgt ggtggcaggc acctgtagtc 50

<210> 43
<211> 50
<212> DNA
<213> Artificial Sequence

<220>
<223> Description of Artificial Sequence:promoter

<400> 43
gactgctcct cttaaagggt gggccctcct cacccagctc cctgccctgg          50


<210> 44
<211> 26
<212> DNA
<213> Artificial Sequence

<220>
<223> Description of Artificial Sequence:oligonucleotide

<400> 44
tgaagtaaaa tcaatatcca accagg          26


<210> 45
<211> 26
<212> DNA
<213> Artificial Sequence

<220>
<223> Description of Artificial Sequence:oligonucleotide

<400> 45
gcactgctcc tcgaactcat gcagcc          26


<210> 46
<211> 26
<212> DNA
<213> Artificial Sequence

<220>
<223> Description of Artificial Sequence:oligonucleotide

<400> 46
tccaaccagg tgtggaagtt ccagcg          26


<210> 47
<211> 26
<212> DNA
<213> Artificial Sequence

<220>
<223> Description of Artificial Sequence:oligonucleotide

<400> 47
ggaagtgctc ctgcacgcac tgctcc          26

<210> 48
<211> 24
<212> DNA
<213> Artificial Sequence

<220>
<223> Description of Artificial Sequence:oligonucleotide

<400> 48
ctggtggaag aagaggacgt ccat                                          24


<210> 49
<211> 24
<212> DNA
<213> Artificial Sequence

<220>
<223> Description of Artificial Sequence:oligonucleotide

<400> 49
tctgggacat acattctgag acat                                          24